# EUROPEAN PATENT APPLICATION

(11) **EP 3 305 893 A1**
(43) Date of publication of application: **11.04.2018**
(21) Application number: 16193002.9
(22) Date of filing: 10.10.2016
(51) Int. Cl.: C12N 9/10, C07K 14/46, C12N 15/82

(54) **PRODUCTION OF POLYSIALYLATED POLYPEPTIDES IN PLANTS AND PLANT CELLS**

(71) Applicant: Universität für Bodenkultur Wien, 1180 Wien (AT)
(72) Inventor: Steinkellner, Hertha, 1190 Wien (AT); Strasser, Richard, 1190 Wien (AT); Castilho, Alexandra, 1080 Wien (AT); Kallolimath, Somanath, 1100 Wien (AT)
(74) Representative: KLIMENT & HENHAPEL

(57) **Abstract**

The present invention relates to a plant or plant cell being capable to produce polysialylated glycoproteins comprising at least one recombinant nucleic acid sequence operably linked to a promoter, said recombinant nucleic acid sequence encoding for a polypeptide lacking a polysialyltransferase binding motif and comprising at least one glycosylation site.

## Description

### Technical Field

The present invention is in the field of glycobiology and protein engineering. More specifically, the present invention relates to polysialylated polypeptides produced in plants and plant cells and to plants and plant cells capable to produce such polypeptides.

### Background Art

Recombinant proteins like monoclonal antibodies (mAbs), hormones, growth factors etc. hold great promise as therapeutic agents against a variety of diseases. However, the efficacy of protein drugs is often compromised by short in vivo half-lives. This limitation arises from susceptibility to proteolytic degradation, immunocomplex formation or clearance from the bloodstream. As a consequence, the efficacy of these drugs depends on frequent administration in large doses leading to high costs and serious side effects.

Efforts have been made to overcome these problems including the conjugation of polymers to the protein to improve the residence time and reduce the immunogenicity. One common modification is the attachment of polyethylene glycol (PEG) which affects the physicochemical features of the protein leading, for example, to improved solubility. While PEGylation of therapeutic proteins can increase the circulating half-life, PEG is not metabolized leading to accumulation in tissues and PEGylated proteins can elicit the formation of unwanted anti-PEG antibodies. Due to the concerns related to the use of PEGylated drugs alternative methods are explored to improve the pharmacokinetic properties of recombinant proteins. The attachment of glycan polymers like polysialic acid to proteins represents another approach to increase the half-life of therapeutic proteins. Polysialic acid has similar physicochemical properties like PEG. In contrast to the synthetic PEG, polysialic acid is naturally occurring in mammals on a small number of proteins, it is biodegradable and nonimmunogenic.

Polysialic acid is either chemically or enzymatically conjugated to amino acids of proteins or to glycans. Both methods require the separate production of the recombinant protein, the polysialic acid (for chemical conjugation) or the polysialyltransferase (for enzymatic conjugation). These in vitro processes are therefore technically challenging and expensive limiting the broadly use of polysialic acids for improving the therapeutic efficacy. Consequently, in vivo generation of polysialic acid on therapeutically relevant recombinant proteins can provide an advantage over existing technologies.

### Summary of invention

The polysialylation of polypeptides comprising a polysialylation domain or motif in plants and plant cells has been recently described by Kallolimath S et al. (PNAS 113(2016):9498-9503; doi:10.1073/pnas.1604371113). However, many proteins and polypeptides, in particular therapeutic polypeptides, lack a polysialylation domain or motif. Therefore, an object of the present invention is the provision of means and methods to polysialylate in vivo recombinant (glyco)proteins or polypeptides lacking a polysialylation domain or motif.

The present invention relates to a plant or plant cell being capable to produce polysialylated glycoproteins comprising at least one recombinant nucleic acid sequence operably linked to a promoter, said recombinant nucleic acid sequence encoding for a polypeptide lacking a polysialyltransferase binding motif and comprising at least one glycosylation site.

It was surprisingly found that plant cells which are able to produce polysialylated glycoproteins (see e.g. Kallolimath S et al. (PNAS 2016, doi:10.1073/pnas.1604371113) can be used to polysialylate polypeptides lacking a polysialyltransferase binding motif and comprising at least one glycosylation site. Such polypeptides are usually not polysialylated in mammalian cells which are known to comprise a polysialylation machinery to polysialylate proteins and polypeptides comprising a polysialyltransferase binding motif.

A further aspect of the present invention relates to a method for producing a polysialylated polypeptide comprising the step of cultivating a plant or plant cell as defined above.

The plants or plant cells of the present invention can be used to produce polysialylated polypeptides lacking a polysialyltransferase binding motif and comprising at least one glycosylation site.

Another aspect of the present invention relates to a polysialylated polypeptide obtainable by a method according to the present invention.

### Brief description of the figures

Fig. 1 shows a schematic presentation of the multi-gene vectors used for leaf disc transformation of Nicotiana benthamiana ΔXTFT.
Fig. 2 shows a schematic presentation of individual binary vectors used to express proteins and enzymes of the sialic acid pathway in Nicotiana benthamiana ΔXTFT including the full length human alpha 2,6-sialyltransferase (ST6) and human alpha2,3-sialyltransferase (ST3).
Fig. 3 shows a schematic presentation of the binary vectors used in the examples to transiently express mammalian polysialyltransferases in Nicotiana benthamiana ΔXTFT and ΔXTFT^{Sia}.
Fig. 4 shows an illustration of the engineered pathway for generation of polysialylated N-glycans in plants.
Fig. 5 shows an illustration of the domain structure of a typical Golgi located type II membrane protein including a CTS region.
Fig. 6 lists examples for CTS regions for targeting and retention of polysialyltransferase in the medial-to-trans Golgi of plants.

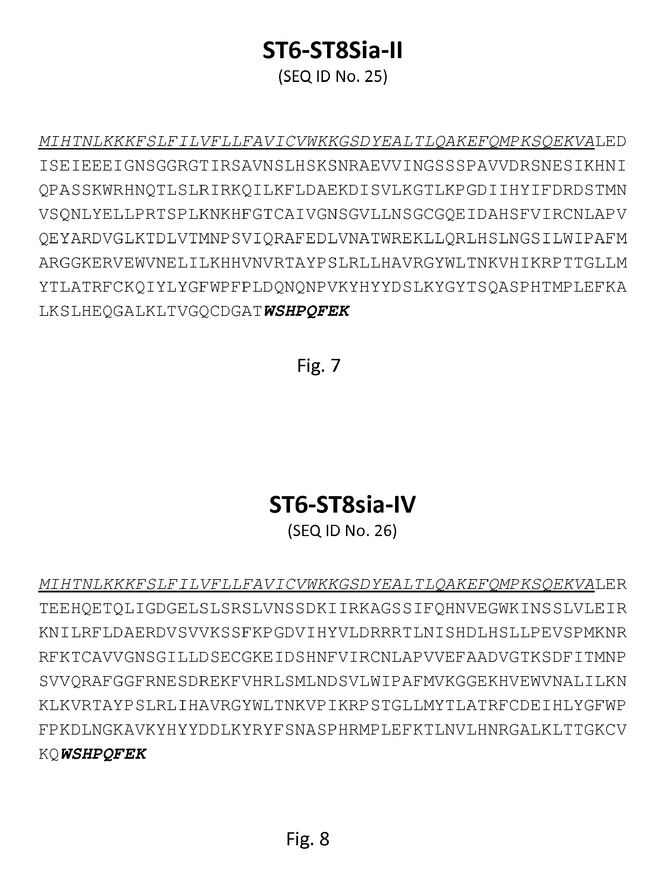

Fig. 7 shows the sequence of the rat ST6 CTS region fused to the catalytic domain of human polysialyltransferase ST8Sia-II as used in vector ST6-ST8Sia-II. The CTS region and the C-terminal strep-tag (WSHPQFEK; SEQ ID No. 39) are shown in underlined/italic and bold/italic letters, respectively.
Fig. 8 shows the sequence of the rat ST6 CTS region fused to the catalytic domain of human polysialyltransferase ST8Sia-IV as used in vector ST6-ST8Sia-IV. The CTS region and the C-terminal strep-tag (WSHPQFEK; SEQ ID No. 39) are shown in underlined/italic and bold/italic letters, respectively.
Fig. 9 shows an immunoblot of protein extracts obtained from plants expressing recombinantly erythropoietin (EPO), fragment crystallizable (Fc), α1-Antitrypsin (A1AT), human transferrin (hTF) and butyrylcholinesterase (BChE) using anti-polySia antibodies.

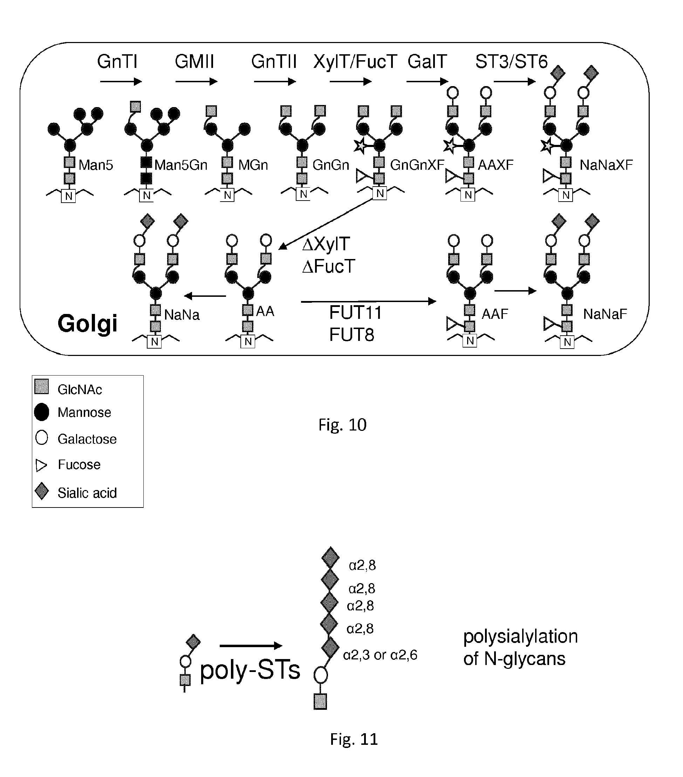

Fig. 10 shows an illustration of the engineering steps leading to sialylated N-glycans that serve as acceptor substrates for polysialylation.
Fig. 11 shows an illustration of the polysialylation reaction on N-glycans.
Fig. 12 shows illustrations of sialylated bi-antennary N-glycan acceptor substrates without (top) or with core fucose (bottom). Illustrations are made according to the symbols from the Consortium for Functional Glycomics (http://www.functionalglycomics.org/). The structures are labelled according to the PROGLYCAN nomenclature (http://www.proglycan.com/). The prefix "iso" denotes the presence of branch isomers.
Fig. 13 shows illustrations of examples for possible sialylated tri- and tetra-antennary N-glycan structures that may serves as acceptors for polysialylation. Additional structures lacking different galactose or GlcNAc residues are possible.
Fig. 14 shows an illustration of the mucin-type O-glycan biosynthesis pathway that needs to be introduced into plants for the generation of sialylated O-glycans.
Fig. 15 lists possible sialylated mucin-type O-glycans that may serve as substrates for polysialylation.
Fig. 16 illustrates the polysialylation reaction on mucin-type O-glycans.
Fig. 17 lists examples for signal peptide sequences that can be used to target polysialyltransferases for secretion to post-Golgi organelles or the apoplast.
Fig. 18 shows an illustration of the expression vector and the sequence of a secreted variant of polysialyltransferase ST8Sia-II (chimeric fusion to the barley alpha-amylase signal peptide sequence). The signal peptide sequence and the C-terminal strep-tag (WSHPQFEK; SEQ ID No. 39) are shown in underlined/italic and bold/italic letters, respectively.
Fig. 19 shows an illustration of the expression vector and the sequence of a secreted variant of polysialyltransferase ST8Sia-IV (chimeric fusion to the barley alpha-amylase signal peptide sequence). The signal peptide sequence and the C-terminal strep-tag (WSHPQFEK; SEQ ID No. 39) are shown in underlined/italic and bold/italic letters, respectively.

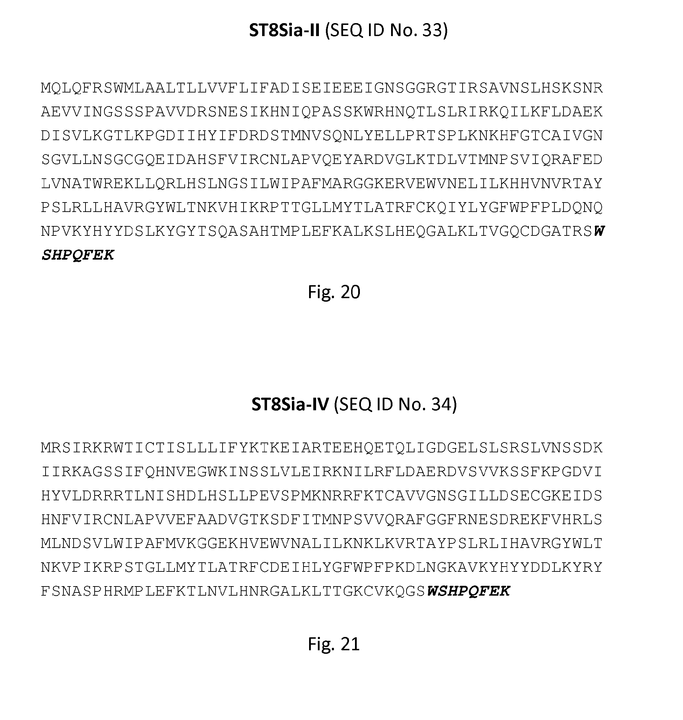

Fig.20 shows the full-length human ST8Sia-II sequence that can be used for polysialylation in plants. The C-terminal strep-tag (WSHPQFEK; SEQ ID No. 39) is shown in bold/italic letters.
Fig.21 shows the full-length human ST8Sia-IV sequence that can be used for polysialylation in plants. The C-terminal strep-tag (WSHPQFEK; SEQ ID No. 39) is shown in bold/italic letters.
Fig. 22 shows an illustration of the expression vector and the sequence of the bacterial polysialyltransferase from N. meningitides (PSTNmB, amino acids 21-496) fused to the CTS region (shown in bold/italic letters) of rat ST6. The shown PSTNmB sequence carries the K69Q mutation as described by Keys et al. (Nature Chem Biol 10(2014):437-442, doi: 10.1038/nchembio.1501).
Fig. 23 shows an illustration of the expression vector and the sequence of the bacterial polysialyltransferase from N. meningitides (PSTNmB, amino acids 21-496) fused to the CTS region of human polysialyltransferase ST8Sia-IV (shown in bold/italic letters). The shown PSTNmB sequence carries the K69Q mutation as described by Keys et al. (Nature Chem Biol, 2014, doi:10.1038/nchembio.1501).

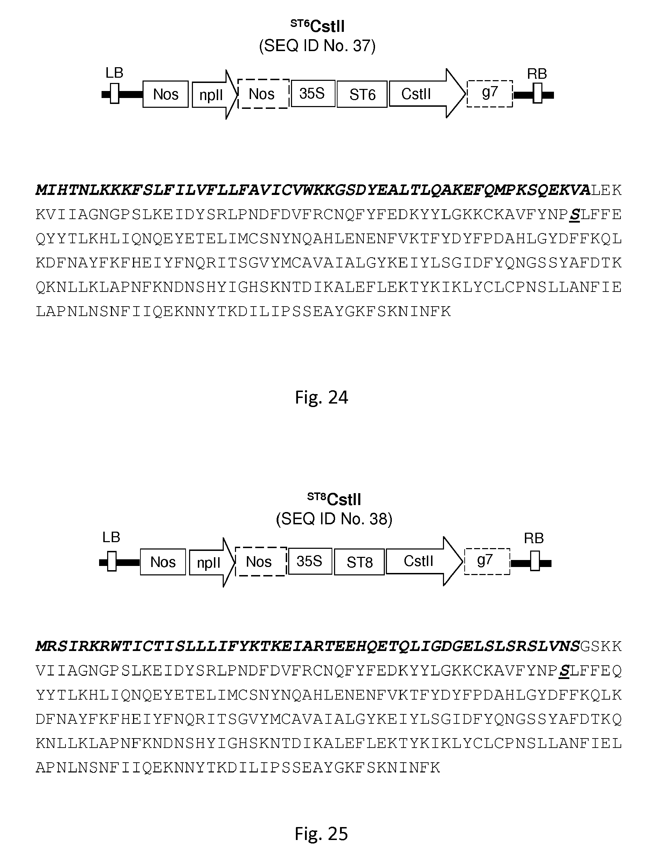

Fig. 24 shows an illustration of the expression vector and the sequence of the bacterial alpha2,3-/alpha2,8-sialyltransferase from Campylobacter jejuni (CstII, amino acids 2-260) fused to the CTS region of rat ST6 (shown in bold/italic letters). The shown CstII sequence carries the I53S mutation as described by Gilbert et al. (J Biol Chem (JBC) 277(2002):327-337, doi: 10.1074/jbc.M108452200).
Fig. 25 shows an illustration of the expression vector and the sequence of the bacterial alpha2,3-/alpha2,8-sialyltransferase from Campylobacter jejuni (CstII, amino acids 2-260) fused to the CTS region of human polysialyltransferase ST8Sia-IV (shown in bold/italic letters). The shown CstII sequence carries the I53S mutation as described by Gilbert et al., (JBC, 2002, doi: 10.1074/jbc.M108452200).

### Description of embodiments

The present invention relates to a plant or plant cell being capable to produce polysialylated glycoproteins comprising at least one recombinant nucleic acid sequence operably linked to a promoter, said recombinant nucleic acid sequence encoding for a polypeptide lacking a polysialyltransferase binding motif and comprising at least one glycosylation site.

It was surprisingly found that a plant or plant cell being capable to produce polysialylated glycoproteins and comprising at least one recombinant nucleic acid sequence operably linked to a promoter, said recombinant nucleic acid sequence encoding for a polypeptide lacking a polysialyltransferase binding motif and comprising at least one glycosylation site, can be used to polysialylate said polypeptide.

A "plant or plant cell being capable to produce polysialylated glycoproteins", as defined herein, refers to plants or parts thereof and plant cells which are able to polysialylate proteins and polypeptides typically comprising a polysialyltransferase binding motif/domain. Since plants and plant cells are known to not sialylate such proteins and polypeptides as plants lack mammalian-type sialic acids as shown for instance by Zeleny et al. (Planta 224(2006):222-227, doi:10.1007/s00425-005-0206-8) nucleic acid molecules encoding enzymes involved in the sialylation and polysialylation of proteins from other organisms, like mammalian or human cells or bacteria, have to be introduced in said plants and plant cells. Enzymes required may include enzymes involved in the biosynthesis of sialic acids and enzymes involved in the attachment of a sialic acid to a core sugar structure present on a protein or polypeptides and the formation of a sialic acid chain thereon. Such plants and plant cells are described, for instance, in Kallolimath S et al. (PNAS 2016, doi:10.1073/pnas.1604371113).

With the plants and plant cells of the present invention polysialylated glycoproteins can be produced which have, i.a., increased half-life time when administered to a mammal compared to a non-polysialylated glycoprotein. One of the major advantages of polysialic acid chains attached to proteins is that these chains are biodegradable and nonimmunogenic whereas PEG does not have these advantages. With the present invention it is now possible to polysialylate proteins and glycoproteins which do not comprise a polysialyltransferase binding motif.

"Polysialylated glycoproteins" or "polysialylated proteins", as used herein, refers to proteins and polypeptides comprising a sugar chain N-linked onto an asparagine residue of a protein or polypeptide. The polysialic acid chain is generated by stepwise transfer of alpha-linked sialic acid added onto a core carbohydrate sequence.

A "polysialic acid chain" or a "polysialic acid" (PSA), as used herein, refers to a glycan chain comprising at least two sialic acid molecules linked alpha-(2-8) and/or alpha-(2-9) to each other.

Polysialic acids (PSAs) are unbranched polymers of sialic acid produced by certain bacterial strains and in mammals in certain cells and on certain proteins. They can be produced in various degrees of polymerization from about 2 to about 400 or more sialic acid molecules. The polysialic acid chain attached to the polysialylated glycoprotein of the present invention comprises preferably sialic acid molecules of, e.g., about 2, about 3, about 4, about 5, about 6, about 7, about 8, about 9, about 10, about 15, about 20, about 25, about 30, about 35, about 40, about 45, about 50, about 75, about 100, about 150, about 200, about 250, about 300, about 350 or about 400. According to another preferred embodiment of the present invention the polysialic acid chain comprises sialic acid molecules of, e.g., at least 2, at least 3, at least 4, at least 5, at least 6, at least 7, at least 8, at least 9, at least 10, at least 15, at least 20, at least 25, at least 30, at least 35, at least 40, at least 45, at least 50, at least 75, at least 100, at least 150, at least 200, at least 250, at least 300, at least 350 or at least 400. According to a further embodiment of the present invention the polysialic acid chain disclosed herein comprises sialic acid molecules of, e.g., at most 2, at most 3, at most 4, at most 5, at most 6, at most 7, at most 8, at most 9, at most 10, at most 15, at most 20, at most 25, at most 30, at most 35, at most 40, at most 45, at most 50, at most 75, at most 100, at most 150, at most 200, at most 250, at most 300, at most 350 or at most 400.

According to a preferred embodiment of the present invention the polysialic acid chain on the glycoprotein of the present invention comprises sialic acid molecules in the range of, e.g., about 2 to about 400, about 2 to about 350, about 2 to about 300, about 2 to about 250, about 2 to about 200, about 2 to about 150, about 2 to about 100, about 2 to about 75, about 2 to about 50, about 2 to about 40, about 2 to about 30, about 2 to about 25, about 2 to about 20, about 2 to about 15, about 2 to about 10, about 5 to about 400, about 5 to about 350, about 5 to about 300, about 5 to about 250, about 5 to about 200, about 5 to about 150, about 5 to about 100, about 5 to about 75, about 5 to about 50, about 5 to about 40, about 5 to about 30, about 5 to about 25, about 5 to about 20, about 5 to about 15, about 5 to about 10, about 10 to about 400, about 10 to about 350, about 10 to about 300, about 10 to about 250, about 10 to about 200, about 10 to about 150, about 10 to about 100, about 10 to about 75, about 10 to about 50, about 10 to about 40, about 10 to about 30, about 10 to about 25, about 10 to about 20, about 10 to about 15, about 50 to about 400, about 50 to about 350, about 50 to about 300, about 50 to about 250, about 50 to about 200, about 50 to about 150, about 50 to about 100, about 50 to about 75, about 100 to about 400, about 100 to about 350, about 100 to about 300, about 100 to about 250, about 100 to about 200, about 150 to about 400, about 150 to about 350, about 150 to about 300, about 150 to about 250, about 150 to about 200, about 200 to about 400, about 200 to about 350, about 200 to about 300 or about 200 to about 250.

The plant and plant cell of the present invention comprise at least one, preferably at least two, more preferably at least three, more preferably at least five, recombinant nucleic acid sequences/molecules which encode for a polypeptide lacking a polysialyltransferase binding motif/domain. In order to allow the biosynthesis of recombinant proteins and polypeptides within a cell the respective nucleic acid sequence has to be operably linked at least to a promoter.

"Recombinant", as used herein, indicates that the cell replicates heterologous nucleic acid molecules or expresses a polypeptide or protein encoded by a heterologous nucleic acid. Recombinant nucleic acid sequences are not found within the native (non-recombinant) form of the cell or plant. A "recombinant polypeptide" is expressed by transcription of a recombinant nucleic acid sequence.

Expression of a polypeptide, as used herein, indicates stable transformation leading to integration of the transgene into the genome as well as transient expression using techniques like agroinfiltration. Respective methods and means to be used in these methods are well known to a person skilled in the art.

As used herein, "operably linked" refers to a functional linkage between a promoter and the nucleic acid molecule encoding the polypeptide of the present invention, wherein the promoter sequence initiates and mediates transcription of the DNA sequence corresponding to said nucleic acid molecule.

The term "promoter", as used herein, refers to a region of a nucleic acid molecule upstream from the start of transcription and involved in recognition and binding of RNA polymerase and other proteins to initiate transcription. Promoters are able to control (initiate) transcription in a cell. Plant promoters are able of initiating transcription in plant cells whether or not its origin is a plant cell. Such promoters include promoters obtained from plants, plant viruses and bacteria which comprise genes expressed in plant cells such Agrobacterium or Rhizobium. The promoter used in the vector of the present invention can be "inducible" or "repressible", i.e. under environmental control. Such promoters can be controlled by changing the cultivation conditions (e.g. temperature) or by adding specific substances. Of course, the promoter used in the vectors of the present invention may be a "constitutive" promoter. Constitutive promoters are active under most environmental conditions and express continuously a protein or polypeptide of interest.

According to a preferred embodiment of the present invention the promoter is selected from the group consisting of promoters active in plants and plant cells, like the cauliflower mosaic virus 35S promoter, opine (octopine, nopaline, etc.) synthase promoters, actin promoter, ubiquitin promoter, etc.

In order to prevent transcriptional activation of downstream nucleic acid sequences by upstream promoters the vector of the present invention may comprise a "terminator" or "terminator sequence". According to a preferred embodiment of the present invention the vector comprises a terminator which is preferably a g7T terminator, a octopine synthase terminator, a manopine synthase terminator, a nopaline synthase or agropine synthase terminator.

"Polysialyltransferase", as used herein, refers to enzymes that are able to produce polysialic acid chains, preferably homopolymers of alpha-2,8-linked sialic acid molecules, homopolymers of alpha-2,9-linked sialic acid molecules or co-polymers of alpha-2,8/alpha-2,9-linked sialic acid molecules on proteins and polypeptides acting as acceptor and using activated nucleotide sugars (uridine, guanosine and cytidine monophosphate derivatives of sugars (UMP, GMP and CMP, respectively) or diphosphate derivatives sugars (UDP, GDP and CDP, respectively)) as donors.

A polypeptide "lacking a polysialyltransferase binding motif" or "lacking a polysialyltransferase binding domain", as used herein, refers to a polypeptide or protein which does not contain a polysialyltransferase binding motif or domain recognized by a polysialyltransferase in an animal cell, preferably a mammalian cell, known to produce polysialylated proteins. A polypeptide "lacking a polysialyltransferase binding motif" or "lacking a polysialyltransferase binding domain" can be simply identified by recombinantly expressing said polypeptide in an animal cell, preferably a mammalian cell, producing polysialylated proteins. If no polysialic acid chains are attached to said polypeptide, the polypeptide can be considered as "lacking a polysialyltransferase binding motif" or "lacking a polysialyltransferase binding domain".

The mammalian polysialyltransferases are active on a limited number of glycoproteins including the neural cell adhesion molecule (NCAM), neuropilin-2, the CD-36 scavenger receptor, the alpha-subunit of the voltage-dependent sodium channel, the synaptic cell adhesion molecule (SynCAM1), the central chemokine receptor CCR7 and on themselves leading to autopolysialylation. Together with the inability of polysialyltransferases to act on free N-glycans, these findings indicate that polysialylation in mammalian cells is a protein-specific modification event requiring initial protein-protein interaction between a polysialyltransferase and its substrate glycoprotein. The first fibronectin type III repeat (FN1) of NCAM is required for binding polysialyltransferases and for polysialylation of N-glycans on the NCAM Ig5 immunoglobulin domain (Thompson et al., JBC 286(2011):4525-4535; DOI 10.1074/jbc.M110.200386). The NCAM FN1 domain represents a polysialyltransferase binding domain. Within this domain an acidic surface patch, an alpha-helix and the QVQ sequence play a role in polysialyltransferase recognition and positioning (Mendiratta et al., JBC 280(2005):32340-32348; DOI 10.1074/jbc.M506217200; Mendiratta et al., JBC 281(2006):36052-36059; DOI 10.1074/jbc.M608073200). A polybasic region within mammalian polysialyltransferases (residues 71-105 in ST8-Sia-IV and residues 86-120 in ST8-Sia-II) interacts with NCAM (Zapater et al., JBC 287(2012):6441-6453, DOI 10.1074/jbc.M111.322024).

The term "plant", as used herein, encompasses plants at any stage of maturity or development, as well as any tissues or organs ("plant parts") taken or derived from any such plant. Plant parts include, but are not limited to, plant cells, stems, roots, flowers, ovules, stamens, seeds, leaves, embryos, meristematic regions, callus tissue, anther cultures, gametophytes, sporophytes, pollen, microspores, protoplasts, hairy root cultures and/or the like. As used herein, a "plant cell" includes, but is not limited to, a protoplast, gamete producing cell, and a cell that regenerates into a whole plant. Tissue culture of various tissues of plants and regeneration of plants therefrom is well known in the art and is widely published.

According to a preferred embodiment of the present invention the plant or plant cell comprises at least one nucleic acid sequence encoding for at least one polysialyltransferase operably linked to at least one promoter.

Polysialyltransferases catalyze the formation of polysialic acid chains by linking sialic acid molecules to each other.

According to a further preferred embodiment of the present invention the at least one polysialyltransferase is a eukaryotic, preferably mammalian, more preferably human, polysialyltransferase or bacterial polysialyltransferase or a variant thereof.

A "variant" of a polysialyltransferase includes molecules having an amino acid sequence that has at least 60%, preferably at least 65%, more preferably at least 70%, more preferably at least 75%, more preferably at least 80%, more preferably at least 85%, more preferably at least 90%, more preferably at least 95%, more preferably at least 97%, more preferably at least 98%, more preferably at least 99%, amino acid sequence identity, preferably over a region of over a region of at least about 75, at least about 100, at least about 200 or at least about 300 amino acid residues, to an amino acid sequence encoded by a naturally occurring polysialyltransferase nucleic acid or to a naturally occurring amino acid sequence of a polysialyltransferase protein.

"Identity", as used herein, refers to two or more sequences or subsequences that are the same or have a specified percentage of amino acid residues or nucleotides that are the same, when compared and aligned for maximum correspondence, as measured using sequence comparison algorithms. It is particularly preferred to use BLAST and BLAST 2.0 algorithms (see e.g. Altschul et al. J. MoI. Biol. 215(1990): 403-410 and Altschul et al. Nucleic Acids Res. 25(1977): 3389-3402) using standard or default parameters. For amino acid sequences, the BLASTP program (see http://blast.ncbi.nlm.nih.gov/Blast.cgi) uses as defaults a wordlength (W) of 6, an expectation (E) of 10 and the BLOSUM62 scoring matrix (see Henikoff and Henikoff, Proc. Natl. Acad. Sci. USA 89(1989):10915) using Gap Costs Existance:11 Extension:1.

The at least one polysialyltransferase is preferably a alpha2,8-polysialyltransferase.

According to a preferred embodiment of the present invention the at least one polysialyltransferase is selected from the group consisting of ST8Sia-II (e.g. GenBank Acc.No. U33551), ST8Sia-IV (e.g. GenBank Acc.No. L41680) and variants thereof.

These mammalian enzymes are Golgi-resident type II membrane proteins composed of a short N-terminal cytoplasmic tail, a single transmembrane domain and a stem region linked to the large catalytic domain which faces the Golgi lumen. Variants include truncated variants of polysialyltransferases lacking N-terminal targeting and retention sequences. Other variants include, for example, mutations near a sialylmotif like the E141K substitution in ST8Sia-II that affects polysialyltransferase activity and results in shorter polysialic acid chains (Isomura et al., JBC 286(2011):21535-21545, DOI 10.1074/jbc.M111.221143).

According to a further preferred embodiment of the present invention a cytoplasmic transmembrane stem (CTS) region of the at least one polysialyltransferase is replaced by a heterologous CTS region, preferably by a plant CTS region.

Particularly preferred plant CTS regions to be used herein include a CTS region from beta1,3-galactosyltransferases 1 (GALT1; Strasser et al., Plant Cell 19(2007):2278-2292; http://dx.doi.org/10.1105/tpc.107.052985), from beta 1,3-galactosyltransferase 3 (GALT3; e.g. Acc.No. At3g06440), from beta1,2-xylosyltransferase (XylT), beta1,2-N-acetylglucosaminyltransferase (GntII) or from plant fucosyltransferases (e.g. FUT11 to FUT13).

According to a further preferred embodiment of the present invention a cytoplasmic transmembrane stem (CTS) region of the at least one polysialyltransferase is replaced by a heterologous CTS region from a mammalian Golgi-resident glycosyltransferase, preferably by the CTS region from rat alpha 2,6-sialyltransferase (ST6), human alpha 2,6-sialyltransferase (ST6) or human alpha2,3-sialyltransferase (ST3).

The exchange of a naturally occurring CTS region of a protein or polypeptide with a CTS region of another polypeptide or protein allows to direct the enzymatic activity of the at least one polysialyltransferase to a specific compartment within a cell. This may have an influence on the polysialylation capability and capacity of the plant or plant cell so that the polysialylation efficiency can be increased.

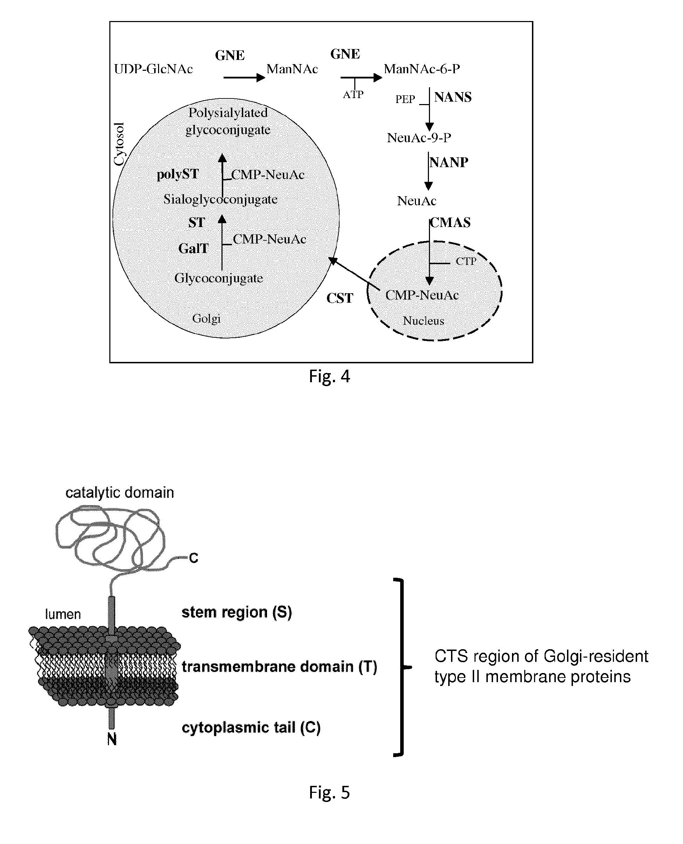

As used herein, a "cytoplasmic transmembrane stem (CTS) region" and a "CTS region" or a "cytoplasmic transmembrane stem (CTS) domain" and a "CTS domain" comprises the cytoplasmic tail, transmembrane domain and stem region of Golgi-resided proteins and polypeptides (see e.g. Fig. 5). CTS regions mediate sorting of the proteins and polypeptides attached thereto into the different functional compartments of the Golgi apparatus.

CTS regions of Golgi-resident proteins can be identified using methods well-known in the art, such as, for example, hydropathy plot analysis and sequence alignments with known CTS regions. A CTS region may consist of a substantial part of a CTS region, such as at least 50% or at least 60% or at least 70% or at least 80% or at least 90% of a CTS region. The CTS region/domain may consist of 1 to 100, preferably 5 to 90, more preferably 10 to 80, more preferably 15 to 70, more preferably 15 to 60, more preferably 20 to 50, more preferably 25 to 45, more preferably 30 to 40, amino acid residues located at the C- or N-terminus of a Golgi-resident protein or polypeptide.

The term "replaced by", as used herein, means that the cytoplasmic transmembrane stem (CTS) region of a wild-type polysialyltransferase is at least partially, preferably entirely, exchanged by a heterologous CTS region, whereby "heterologous" means that the CTS region is not naturally occurring in said wild-type polysialyltransferase. Thus, also CTS regions or domains of a polysialyltransferase of another organism are considered as heterologous.

In addition to CTS regions from Golgi resident enzymes, the use of N-terminal membrane anchoring sequences for post-Golgi targeting and retention (e.g. to the Trans Golgi Network) or to other post-Golgi organelles may be beneficial and used to replace the CTS region from the polysialyltransferases.

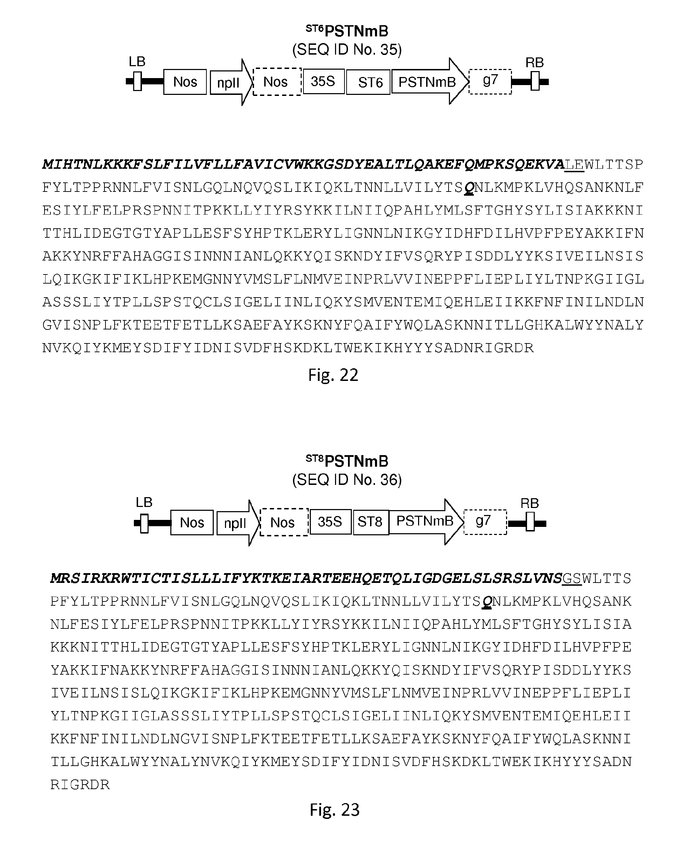

According to a preferred embodiment of the present invention the plant or plant cell of the present invention may be able to express one or more bacterial polysialyltransferases. Bacterial polysialyltransferases do not contain any cytoplasmic tail and transmembrane domain region. Thus, it is preferred that a heterologous CTS region is attached to such bacterial polysialyltransferases (see e.g. Figs. 22 and 23).

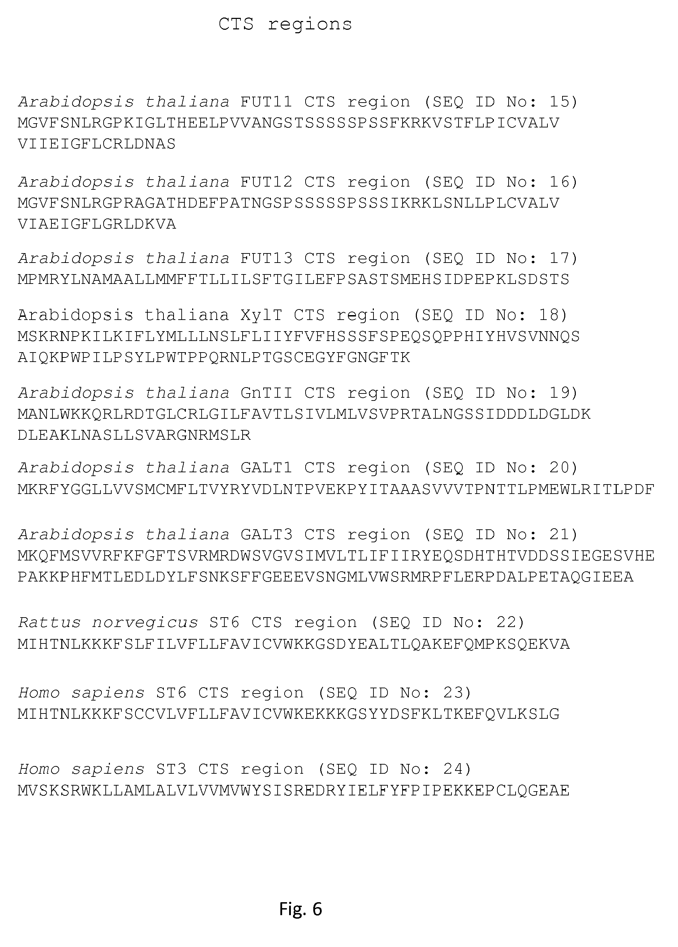

According to a particularly preferred embodiment of the present invention the heterologous CTS region is selected from the group consisting of SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23 and SEQ ID No. 24 (see also Fig. 6).

Preferably, the bacterial polysialyltransferase is selected from the group of gram-negative bacteria including Neisseria meningitides, Escherichia coli, Mannheimia haemolytica, Pasteurella haemolytica and Moraxella nonliquefaciens. According to a preferred embodiment of the present invention the bacterial polysialyltransferase is from N. meningitides serogroup B PSTNmB (e.g. GenBank: AAA20478.1) and may carry an amino acid substitution (K69Q) that alters the size distribution of the product.

According to a further preferred embodiment of the present invention the plant or plant cell comprises at least one nucleic acid sequence encoding for at least one bifunctional bacterial alpha2,3-/alpha2,8-sialyltransferase CstII (GenBank: AAL36462.1) from Campylobacter jejuni, which enables the plant or plant cell to generate disialic acid containing glycoproteins. Glycans with disialic acid can serve as substrates for the generation of polysialic acids. In addition, disialic acids may also increase the half-life of therapeutic proteins because they can slow down the stepwise removal of terminal monosaccharides that is required for binding to specific lectin receptors and subsequent clearance.

According to a further preferred embodiment the bacterial CstII amino acid sequence carries the I53S substitution and a C-terminal truncation (Gilbert et al., JBC, 2002, doi: 10.1074/jbc.M108452200; Chiu et al., Nat Struct Mol Biol. 11(2004):163-170, doi:10.1038/nsmb720; Lindhout et al., PNAS 108(2011):7397-7402, doi: 10.1073/pnas.1019266108; Cheng et al., Glycobiology 18(2008):686-697; doi: 10.1093/glycob/cwn047) to alter the enzyme activity.

According to a further preferred embodiment the disialic acid can be further elongated using bacterial or mammalian polysialyltransferases to form oligosialic or polysialic acid polymers.

According to a further preferred embodiment the formation of the disialic acid is increased by combined expression with other bacterial or mammalian polysialyltransferases.

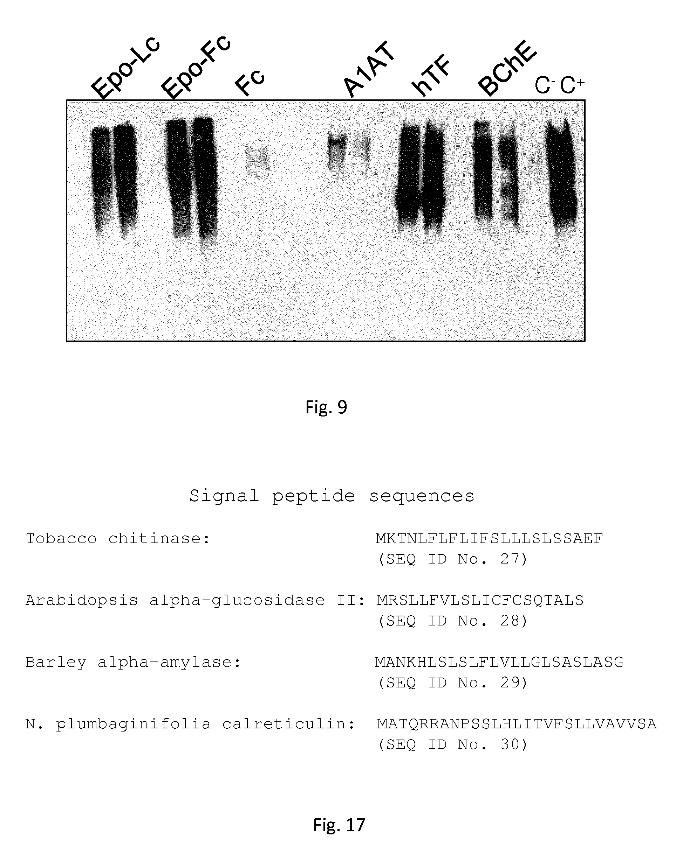

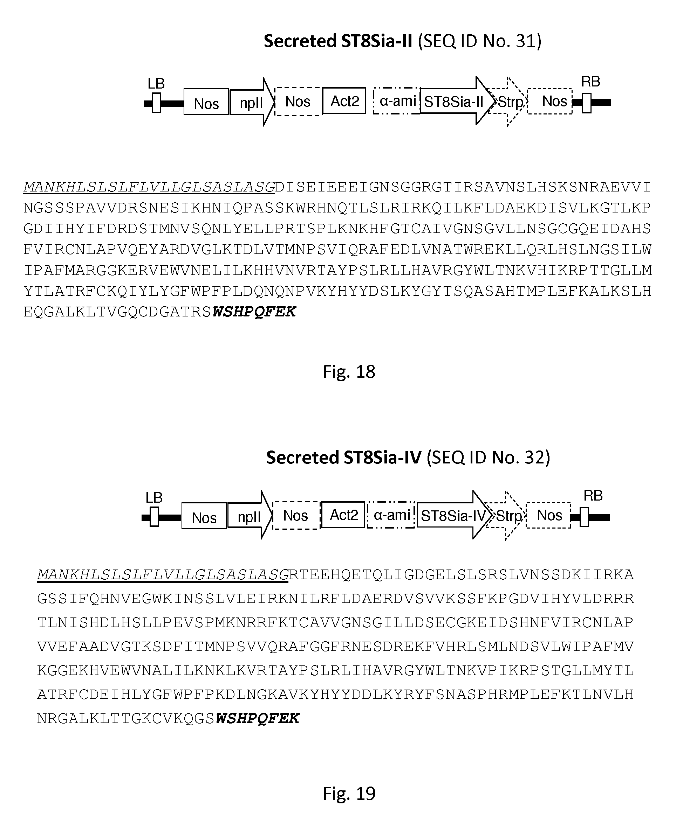

According to a further preferred embodiment of the present invention the N-terminal cytoplasmic tail and transmembrane domain of the at least one polysialyltransferase, preferably mammalian polysialyltransferase, are replaced by a signal peptide sequence (see e.g. Figs. 18 and 19). This replacement prevents intracellular retention and targets the enzyme for secretion to the apoplast (extracellular space). The signal peptide sequences (see e.g. Fig. 17) are preferably from plants including, for example, the signal peptide sequence from barley alpha-amylase.

According to a further preferred embodiment of the present invention a signal peptide sequence for secretion to the apoplast is attached to a bacterial polysialyltransferase.

In order to facilitate expression of all recombinant proteins and polypeptides within the plant and plant cell codon-optimized variants of the respective nucleic acid sequences are introduced into the plant or plant cell. In particular, nucleic acid molecules encoding for bacterial sialyltransferases are codon-optimized. Methods for codon optimization are well known in the art. According to a further preferred embodiment of the present invention the plant or plant cell comprises nucleic acid sequences encoding for enzymes involved in the synthesis of a sialic acid precursor operably linked to at least one promoter.

The polysialylation of polypeptides and proteins requires the presence of sialic acid, in particular of sialic acid precursors, within a plant or plant cell. In order to enable a plant and plant cell to produce sialic acids the plant or plant cell may comprise nucleic acid sequences which encode for proteins that are involved in the synthesis of a sialic acid precursor. These nucleic acid sequences may be recombinantly introduced into a plant or plant cell.

The sialic acid precursor produced within the plant or plant cell is preferably N-acetylneuraminic acid (Neu5Ac), preferably CMP-N-acetylneuraminic acid (CMP-Neu5Ac), or N-glycolylneuraminic acid (Neu5Gc), preferably CMP-N-glycolylneuraminic acid (Neu5Gc). These enzymes and the nucleic acid sequences encoding said enzymes are preferably of mammalian, more preferably of human, origin.

According to a preferred embodiment of the present invention the plant or plant cell comprises at least one nucleic acid sequence encoding for at least one enzyme involved in the synthesis of a sialic acid precursor, wherein the enzymes are preferably selected from the group consisting of UDP-GlcNAc 2-epimerase/N-acetylmannosamine kinase (GNE), N-acetylneuraminic acid phosphate synthase (NANS), CMP-sialic acid synthetase (CMAS) and variants thereof. These enzymes and the nucleic acid sequences encoding said enzymes are preferably of mammalian, more preferably of human, origin and may comprise or consist of the amino acid sequences deposited under UniProtKB Acc.Nos. Q9Y223 (human GNE), Q91WG8 (murine GNE), Q9NR45 (human NANS) and Q8NFW8 (human CMAS).

According to a further preferred embodiment of the present invention the plant or plant cell comprises at least one nucleic acid sequence encoding for at least one enzyme involved in the synthesis of a sialic acid precursor, wherein the mammalian GNE enzyme comprises a mutation at arginine position 263, preferably a R263L mutation to prevent feedback inhibition (see Kallolimath S et al.; PNAS 2016, doi:10.1073/pnas.1604371113).

According to a further preferred embodiment of the present invention genes encoding beta 1,2-xylosyltransferase (XylT; e.g. GenBank Acc.No.EF562628) and/or core alpha 1,3-fucosyltransferase (FucT; e.g. GenBank Acc.No.EF562630) and/or beta-hexosaminidases (HEXOs; e.g. GenBank Acc.No. KX192074) and/or beta 1,3-galactosyltransferases (GALTs; e.g. GenBank Acc.No.NM_001332728) and/or alpha 1,4-fucosyltransferase (e.g. GenBank Acc. No.NM_105857) occurring in the plant or plant cell are mutated, silenced or inhibited to reduce their enzymatic activity within said plant or plant cell.

In order to reduce or even to abolish the formation of plant specific N-glycans (glycan chains attached to an asparagine of a polypeptide or protein) one or more of the genes encoding the above mentioned enzymes are mutated, silenced (e.g. using siRNA or RNAi) or inhibited in the plant or plant cell. As a consequence thereof the respective plants and plant cells are not able to produce N-glycans comprising plant specific beta 1,2-xylose and core alpha 1,3-fucose in an extent as the corresponding wild-type plants and plant cells. Likewise, the formation of N-glycans with Lewis a-type elongations (Fuc alpha1,4-(Gal beta1,3-)GlcNAc) will be prevented or reduced. The mutation may include deletion or substitution of the respective gene(s) or parts thereof (e.g. promoter, coding region), whereby deletion is most preferred. Alternatively, insertions within the respective genes that cause a frame shift in the open reading frame or substitutions of nucleotides leading to nonsense or missense mutations may be used. According to a further preferred embodiment such mutations are carried out using targeted genome editing technologies including CRISPR/Cas9, TALENs or Zinc finger nucleases (Vazquez-Vilar et al., Plant Methods 12(2016):10; doi: 10.1186/s13007-016-0101-2; Li et al., Plant Biotechn. J. 14(2016):533-542, doi: 10.1111/pbi.12403) According to a further preferred embodiment the formation of plant specific N-glycans is abolished by sequence specific targeting of mRNAs (gene silencing approaches using hairpin constructs, antisense sequences, RNAi technology, artificial microRNAs, virus-induced gene silencing and the like). An example for gene silencing using a hairpin construct is shown in Strasser et al. (Plant Biotechn. J 6(2008):392-402, doi: 10.1111/j.1467-7652.2008.00330).

According to a further preferred embodiment the amount of complex N-glycans carrying terminal GlcNAc that is used as acceptor substrate for further elongations with beta 1,4-linked galactose and subsequently with sialic acid is increased by inhibition or inactivation of beta-hexosaminidases from plants. Three different types of beta-hexosaminidases are present in plants, inactivation of beta-hexosaminidase 3 (HEXO3; e.g. GenBank Acc.No.KX192074) has been shown to increase the amounts of complex N-glycans with terminal GlcNAc residues at both branches of secreted recombinant glycoproteins (Castilho et al., Plant Physiol. 166(2014):1839-1851, doi: 10.1104/pp.114.250720; Shin et al., Plant Biotechn. J. 2016, doi: 10.1111/pbi.12602).

The plant or plant cell comprises preferably nucleic acid sequences encoding for beta1,4-galactosyltransfease (GalT; e.g. GenBank Acc.No. X55415), CMP-sialic acid transporter (CST; e.g. GenBank Acc.No.D87969), alpha2,6-sialyltransferase (ST6; e.g. GenBank Acc.No.M18769), alpha2,3-sialyltransferase (ST3; e.g. GenBank Acc.No. L23767) and/or variants thereof operably linked to at least one promoter. These enzymes and the nucleic acid sequences encoding said enzymes are preferably of mammalian, more preferably of human, origin.

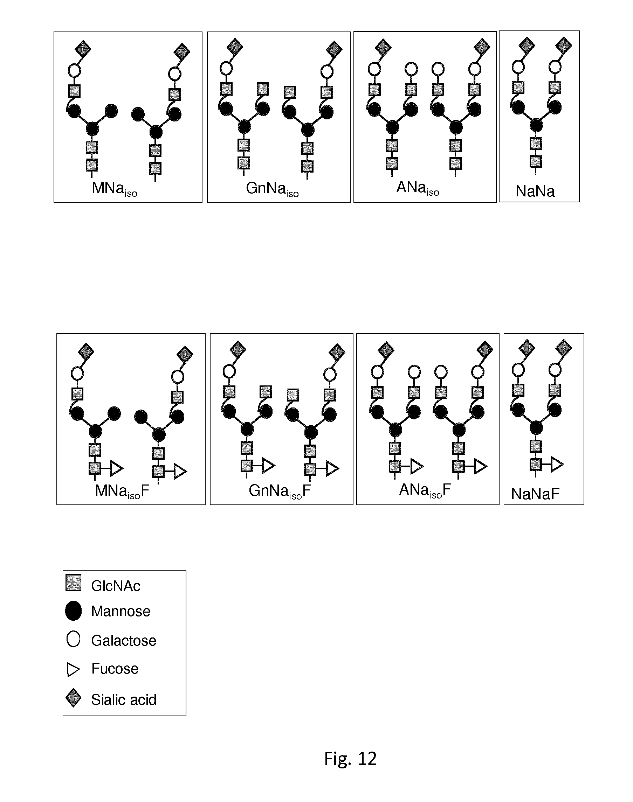

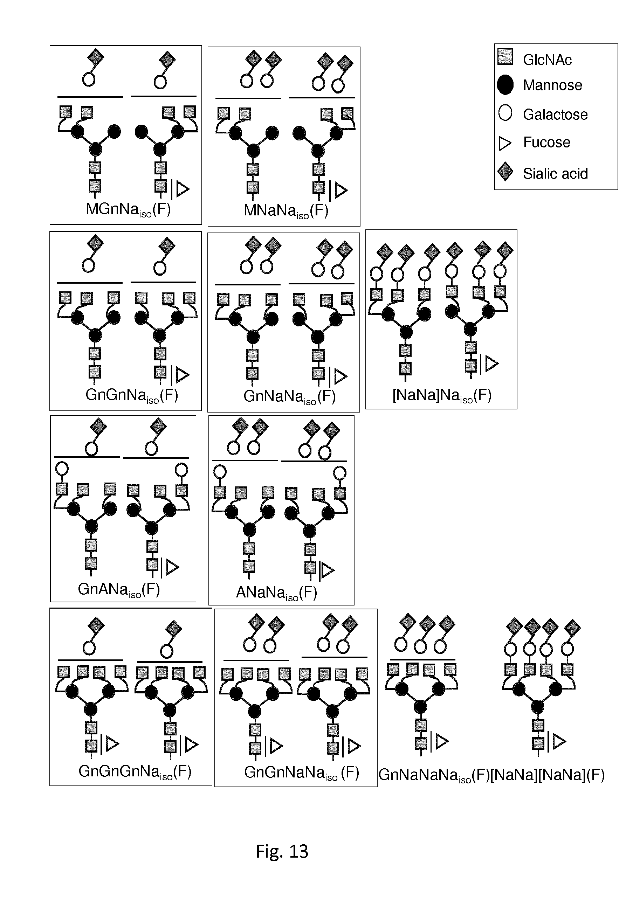

The polysialic acid chain is produced by attaching single sialic acid molecules to each other. This process involves polysialyltransferases. However, these polysialyltransferase are usually not able to attach the first sialic acid of the chain to any acceptor. Therefore, the plant and plant cells are preferably able to produce the aforementioned enzymes to form those structures which form the core region of the N-glycan comprising the polysialic acid chain. Particularly preferred core regions comprise the following structures: di-antennary: NaNa, ANaᵢₛₒ, GnNaᵢₛₒ, MNaᵢₛₒ (see Fig. 12); core fucosylated structures (core alpha 1,3-fucose or core alpha 1,6-fucose): NaNaF, ANaᵢₛₒF, GnNaᵢₛₒF, MNaᵢₛₒF (see Fig. 12); tri-antennary and tetra-antennary complex N-glycans with or without core fucose (see Fig. 13).

According to another preferred embodiment of the present invention the plant or plant cell comprises a nucleic acid sequence encoding for at least one fucosyltransferase, preferably an alpha 1,6-fucosyltransferase (FUT8; e.g. GenBank Acc.No.NM_178155), operably linked to at least one promoter. This enzyme and the nucleic acid sequence encoding said enzyme is preferably of mammalian, more preferably of human, origin.

Mammalian and in particular human glycoproteins contain usually fucose residues linked alpha 1,6 to the first GlcNAc residue of their N-glycans. Alpha 1,6-fucosyltransferase directs the addition of fucose to asparagine-linked GlcNAc moieties.

According to a preferred embodiment of the present invention the plant or plant cell comprises a nucleic acid sequence encoding for at least one N-acetylglucosaminyltransferase, preferably a beta 1,6-N-acetylglucosaminyltransferase (GnTV; e.g. GenBank Acc.No. NM_002410) or a beta 1,4-N-acetylglucosaminyltransferase (GnTIV; e.g. GenBank Acc.No. NM_012214) or a beta 1,2-N-acetylglucosaminyltransferase (GnTII; e.g. GenBank Acc.No. NM_002408) operably linked to at least one promoter. These enzymes and the nucleic acid sequences encoding said enzymes are preferably of mammalian, more preferably of human, origin. GnTIV and GnTV will generate tri- and tetra-antennary N-glycans (also termed branched N-glycans) (Castilho et al., Glycobiology 21(2011):813-823; doi:10.1093/glycob/cwr009) and provide additional terminal GlcNAc residues at the non-reducing end that can be further extended with beta 1,4-galactose, alpha2,3/alpha2,6-liked sialic acid (Castilho et al., Plos One 8(2013):e54836, doi:10.1371/journal.pone.0054836) and serve as acceptor for the attachment of polysialic acid. GnTII is also present in plants. However, on some recombinant glycoproteins, endogenous plant GnTII is not capable to modify all N-glycans very efficiently resulting in mono-antennary N-glycans (Dicker et al., Front Plant Sci. 29(2016):18, doi:10.3389/fpls.2016.00018). Heterologous expression of an animal GnTII enzyme, preferably human GnTII, in such plants will increase the amount of processed complex N-glycans and thus the potential acceptor glycan substrates for polysialylation.

It is particularly preferred that the polysialic acid chain is linked to the polypeptide or protein via a mannose comprising core sugar. In order to attach GlcNAc residues on mannose, for instance, N-acetylglucosaminyltransferases are required.

According to a preferred embodiment of the present invention the plant or plant cell comprises a nucleic acid sequence encoding for at least one core alpha1,3-fucosyltransferase operably linked to at least one promoter. This enzyme and the nucleic acid sequence encoding said enzyme is preferably of plant (Arabidopsis thaliana, maize, etc.), insect, nematode, trematode or snail origin. It has been shown that the presence of core alpha 1,3-fucose can facilitate more efficient sialylation of recombinant glycoproteins when expressed in plants (Castilho et al, mAbs 7(2015):863-870, DOI: 10.1080/19420862.2015.1053683). More efficient sialylatation (capping of terminal beta 1,4-galactose) is beneficial as it will provide more acceptor substrates for polysialylation.

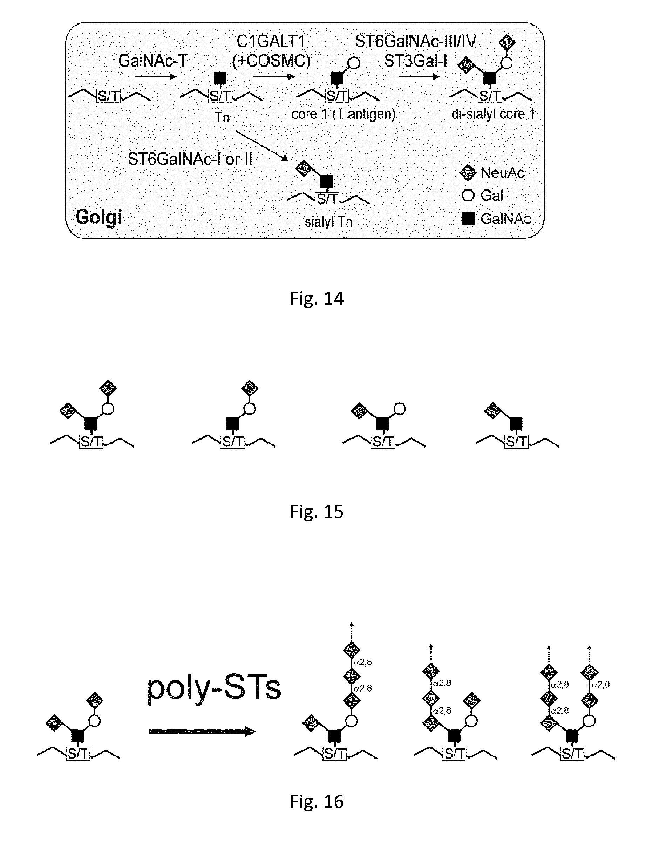

According to a preferred embodiment of the present invention the plant or plant cell comprises a nucleic acid sequence encoding at least one polypeptide:N-acetylgalactosaminyltransferase (GalNAc-T; e.g. GenBank Acc.No.NM_003774), preferably a human GalNAc-T (e.g. GenBank Acc.No. BC041120) for initiation of mucin-type O-glycan biosynthesis. Mucin-type O-glycans are another type of acceptor substrates that can be used by polysialyltransferases to polysialylate polypeptides. NCAM and dendritic cell neuropolin-2 (Foley et al., JBC 285(2010):35056-35067, doi: 10.1074/jbc.M110.170209; Rollenhagen et al., JBC 288(2013):22880-22892, doi:10.1074/jbc.M113.463927; Bhide et al., JBC 291(2016):9444-9457, DOI 10.1074/jbc.M116.714329) can carry polysialylated mucin type O-glycans. Mucin-type O-glycans are not present in plants. In order to initiate the formation and elongate the O-glycans a GalNAc-T and other animal glycosyltransferases have to be recombinantly expressed in plants (see Fig. 14). Core 1 O-glycan synthesis requires, for example, human GalNAc-T2 and Drosophila melanogaster core 1 beta 1,3-galactosyltransferase. Sialylation of core 1 structures can be achieved by expression of mammalian alpha 2,3-sialyltransferase(ST3Gal-I; e.g. GenBank Acc.No.BC018357) and alpha 2,6-sialyltransferase (ST6GalNAc-III; e.g. GenBank Acc.No.BC086784) (Castilho et al., JBC 287(2012):36518-36526, DOI 10.1074/jbc.M112.402685; Dicker et al., Front Plant Sci. Plant, 2016, doi:10.3389/fpls.2016.00018). Alternatively, O-linked GalNAc-residues can also be directly sialylated using an alpha 2,6-sialyltransferase (ST6GalNAc-I e.g. GenBank Acc.No. NM_018414 or ST6GalNAc-II e.g. GenBank Acc.No.NM_006456) (Dicker et al., Bioengineered, 2016, http://dx.doi.org/10.1080/21655979.2016.1201251). These O-glycan engineering approaches result in different O-linked glycan structures (see Fig. 15) that serve as acceptor substrates for polysialylation (see Fig. 16). In addition to sialylated GalNAc and mono- or disialylated core 1 structures, also sialylated core 2 or other extended or branched sialylated mucin-type O-glycans can serve as acceptor substrates for polysialylation. The plant and plant cells are preferably able to produce the aforementioned enzymes to form those mucin-type O-glycan structures with a GalNAc-residue linked to a mammalian O-glycosylation site. A mammalian O-glycosylation site can be simply identified by expression of the polypeptide in a mammalian cell and analysis of the attachment of GalNAc residues as well as further elongations.

According to a further preferred embodiment of the present invention an N-linked trisaccharide consisting of Neu-Ac-Hexose-HexNAc is used as acceptor substrate for polysialyltransferases. Particularly, the trisaccharide structure is Neu5Ac-alpha2,3-galactose-beta1,4-GlcNAc or Neu5Ac-alpha2,6-galactose-beta1,4-GlcNAc. To generate this N-linked acceptor, a nucleic acid sequence encoding for at least one endo-beta-N-acetylglucosaminidase operably linked to at least one promoter is recombinantly expressed in plants. Preferably, the endo-beta-N-acetylglucosaminidase is an endo T as described for mammalian cells (Meuris et al., Nature Biotechnology 32(2015):485-489, doi:10.1038/nbt.2885) and plants (Piron et al., Nature Biotechnology 33(2015):1135-1137, doi:10.1038/nbt.3359). The resulting N-linked GlcNAc is extended by recombinant expression of beta 1,4-galactosyltransferase and alpha 2,3- or alpha 2,6-sialyltransferase resulting in the generation of a (mono)sialylated trisaccharide that serves as acceptor for polysialylation.

According to a further preferred embodiment of the present invention the polysialyltransferase binding motif is a fibronectin type III domain or a fragment thereof like the FN1 acidic patch, preferably an acidic batch including the core acidic residues Asp520, Glu521, and Glu523 (present in the DEPE motif).

The glycosylation site is preferably a N-glycosylation site (Asn-X-Ser/Thr, where X can be any amino acid except proline) or a mucin-type O-glycosylation site (GalNAc linked to Ser/Thr).

The polysialic acid chain and its core structure are attached to a protein or polypeptide via an asparagine, serine or threonine residue.

The polypeptide lacking a polysialyltransferase binding motif is preferably selected from the group consisting of antibodies, like IgG, IgA, IgM, IgD, IgE and fragments thereof including single chain antibodies (scFvs), heavy chain antibodies, Fab-fragments, nanobodies, Fcabs and similar truncated or engineered antibody formats. For instance, small antibody fragments or variants like single chain Fv (ScFv) fragments are rapidly cleared from the blood. Polysialylation of such antibody fragments can increase the in vivo circulating half-life (Chen et al., Bio-conjugate Chem 23(2012):1524-1533, dx.doi.org/10.1021/bc200624a). Another important class of polypeptides lacking polysialyltransferase motifs represents immunoglobulins of the IgA type including monomeric, dimeric and secretory variants as well as the subclasses IgA1 and IgA2.
According to a preferred embodiment of the present invention the polypeptide lacking a polysialyltransferase binding domain is selected from the group consisting of antigen-binding non-immunoglobulin proteins or scaffolds including designed ankyrin repeat proteins (DARPins) or affibodies.

According to a preferred embodiment of the present invention the polypeptide lacking a polysialyltransferase binding domain is selected from the group consisting of erythropoietin, α1-Antitrypsin, transferrin, butyrylcholinesterase, granulocyte colony-stimulating factor, DNAse 1, clotting factors, in particular factor VII, factor VIII, factor IX or von Willebrand factor, follicle-stimulating hormone, luteinizing hormone, thyroid-stimulating hormone, interferons, in particular interferon alpha, interferon beta or interferon gamma, tumor necrosis factor-alpha inhibitors, in particular etanercept, viral proteins, viral antigens, and fragments, mutants or variants thereof.

According to a preferred embodiment of the present invention the polypeptide lacking a polysialyltransferase binding domain is selected from the group consisting of insulin or other non-glycosylated protein therapeutics. To facilitate polysialylation of such proteins (including also non-glycosylated antibody fragments and non-glycosylated non-immunoglobulin scaffolds) an N-glycosylation site (Asn-X-Ser/Thr) or Ser/Thr O-glycosylation site is introduced by site-directed mutagenesis or by insertion or attachment of amino acid residues, small peptides or protein domains.

According to another preferred embodiment of the present invention the plant is selected from the group consisting of the genera Nicotiana, Arabidopsis, Lemna, Physcomitrella, Zea, Oryza, Triticum, Pisum, Lotus, Taxus and Brassica or selected from the group consisting of algae safflower, alfalfa, lettuce, barley, rapeseed, soybean, sugar beet, sugar cane, potato, tomato, spinach, ginseng, gingko and carrots and the plant cell is derived from said plants.

Particularly preferred are plants and plant cells of the genera Nicotiana, Arabidopsis or Oryza.

According to a further preferred embodiment of the present invention the plant is selected from the group of plant species consisting of Nicotiana benthamiana, Nicotiana tabacum, Arabidopsis thaliana, Lemna minor, Physcomitrella patens, Zea mays, Oryza sativa, Triticum aestivum, Pisum sativum, Lotus japonicas, Taxus cuspidate, and Brassica napus.

Particularly preferred are plants and plant cells of Nicotiana benthamiana, Nicotiana tabacum or Arabidopsis thaliana.

According to a preferred embodiment of the present invention the plant cell is selected from the group consisting of tobacco BY2 cells, medicago cells, carrot cells and rice cells.

The plant cell of the present invention and used in the methods of the present invention can be derived from the above mentioned plants, plant genera and plant species. The cells may be derived from any part of these plants.

However, it is particularly preferred that the plant cell is a cambial meristematic cell.

Another aspect of the present invention relates to a method for producing a polysialylated polypeptide comprising the step of cultivating a plant or plant cell according to the present invention.

The plants or plant cells of the present invention can be used to produce polysialylated polypeptides or proteins. Thereby these plants and plant cells are cultivated with methods as described for Nicotiana benthamiana plants (Chen et al., Adv Tech Biol Med 1(2013):103, http://dx.doi.org/10.4172/atbm.1000103) or Arabidopsis thaliana (Arabidopsis Protocols, Methods in Molecular Biology, Volume 1062, 2014, DOI 10.1007/978-1-62703-580-4).

According to a preferred embodiment of the present invention the plant cell is cultivated in suspension culture as described, for example, for tobacco BY2 cells (Nagata et al.,Int. Rev. Cytol. 1992, DOI: 10.1016/S0074-7696(08)62452-3)

Plant cell cultures can be grown as cell suspension cultures in a liquid medium or as callus cultures on a solid medium. Sterile explants are usually placed on the surface of a sterile solid culture medium, but can also be placed directly into a sterile liquid medium, particularly when a cell suspension culture is desired. Explants can be taken from different parts of a plant, including portions of shoots, leaves, stems, flowers, roots, single undifferentiated cells and from many types of mature cells provided are they still contain living cytoplasm and nuclei and are able de-differentiate and resume cell division. Plant cells are, however, preferably cultivated in cell suspension. Suspension cell cultures have several advantages over conventional isolation of products from the intact plants, such as stable supply, freedom from disease and vagaries of climates, closer relationship between supply and demand, and growth of large amount of plant cells in minimal space.

According to a preferred embodiment of the present invention the nucleic acid sequences are introduced into the plant or plant cell by agroinfiltration of the plant cell, plants or parts thereof including leaves, in order to transiently express the polypeptides encoded by said nucleic acid sequences.

A further aspect of the present invention relates to a polysialylated polypeptide obtainable by a method according to the present invention.

The polysialylated polypeptide or protein of the present invention has a unique glycan structure because these polypeptides and proteins are usually not polysialylated by mammalian cells or any other cells since they lack a polysialyltransferase binding motif/domain. Furthermore, the glycan chains comprising the polysialic acid chains comprise a core structure which is usually found in naturally occurring glycoproteins.

According to a preferred embodiment of the present invention the polysialylated polypeptide comprises a polysialic acid chain comprising at least 2, preferably at least 4, more preferably at least 8, sialic acid units. The length of the polysialic acid chain may be influenced by modulating the acceptor substrate binding pocket of the polysialyltransferases. Changes in single amino acid residues (for instance K69Q, H78L and N100I) of the bacterial polysialyltransferase from Neisseria meningitides serogroup B resulted in altered product length (Keys et al., Nature Chem Biol, 2014, doi:10.1038/nchembio.1501). A mutant variant of human ST8Sia-II (E141K substitution) affects polysialyltransferase activity resulting in shorter polysialic acid chains (Isomura et al., JBC, 2011, DOI 10.1074/jbc.M111.221143).

According to a further preferred embodiment of the present invention the polysialylated polypeptide comprises a polysialic acid chain comprising 2 to 400, preferably 2 to 300, more preferably 2 to 250, sialic acid units.

Another aspect of the present invention relates to the use of a plant or plant cell according to the present invention for producing a polysialylated polypeptide from a polypeptide lacking a polysialyltransferase binding motif and comprising at least one glycosylation site.

The present invention is further illustrated by the following embodiments and examples, however, without being restricted thereto.

### Embodiments:

1. Plant or plant cell being capable to produce polysialylated glycoproteins comprising at least one recombinant nucleic acid sequence operably linked to a promoter, said recombinant nucleic acid sequence encoding for a polypeptide lacking a polysialyltransferase binding motif and comprising at least one glycosylation site.
2. Plant or plant cell according to embodiment 1, wherein the plant or plant cell comprises at least one nucleic acid sequence encoding for at least one polysialyltransferase operably linked to at least one promoter.
3. Plant or plant cell according to embodiment 2, wherein the at least one polysialyltransferase is a eukaryotic, preferably mammalian, more preferably human, polysialyltransferase or bacterial polysialyltransferase or a variant thereof.
4. Plant or plant cell according to embodiment 2 or 3, wherein the at least one polysialyltransferase is a alpha2,8-polysialyltransferase.
5. Plant or plant cell according to any one of embodiments 2 to 4, wherein the at least one polysialyltransferase is selected from the group consisting of ST8Sia-II, ST8Sia-IV and variants thereof.
6. Plant or plant cell according to any one of embodiments 2 to 5, wherein a cytoplasmic transmembrane stem (CTS) region of the at least one polysialyltransferase is replaced by a heterologous CTS region, preferably by a plant CTS region.
7. Plant or plant cell according to embodiment 6, wherein the heterologous CTS region is selected from the group consisting of SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23 and SEQ ID No. 24 (see also Fig. 6)
8. Plant or plant cell according to any one of embodiments 2 to 5, wherein a cytoplasmic transmembrane stem (CTS) region of the at least one polysialyltransferase is replaced by a signal peptide sequence, preferably by a plant signal peptide sequence.
9. Plant or plant cell according to any one of embodiments 1 to 8, wherein the plant or plant cell comprises nucleic acid sequences encoding for enzymes involved in the synthesis of a sialic acid precursor operably linked to at least one promoter.
10. Plant or plant cell according to embodiment 9, wherein the sialic acid precursor is N-acetylneuraminic acid (Neu5Ac), preferably CMP-N-acetylneuraminic acid (CMP-Neu5Ac), or N-Glycolylneuraminic acid (Neu5Gc), preferably CMP-N-Glycolylneuraminic acid (Neu5Gc).
11. Plant or plant cell according to any one of embodiments 1 to 10, wherein the plant or plant cell comprises at least one nucleic acid sequence encoding for at least one enzyme involved in the synthesis of a sialic acid precursor, wherein the enzymes are preferably selected from the group consisting of UDP-GlcNAc 2-epimerase/N-acetylmannosamine kinase (GNE), N-acetylneuraminic acid phosphate synthase (NANS), CMP-sialic acid synthetase (CMAS) and variants thereof.
12. Plant or plant cell according to any one of embodiments 1 to 11, wherein genes encoding beta 1,2-xylosyltransferase (XylT) and/or core alpha 1,3-fucosyltransferase (FucT) and/or beta-hexosaminidases (HEX-Os) and/or beta 1,3-galactosyltransferases (GALTs) and/or alpha 1,4-fucosyltransferase occurring in the plant or plant cell are mutated, silenced or inactivated to reduce their enzymatic activity within said plant or plant cell.
13. Plant or plant cell according to any one of embodiments 1 to 12, wherein the plant or plant cell comprises nucleic acid sequences encoding for beta 1,4-galactosyltransfease (GalT), CMP-sialic acid transporter (CST), alpha 2,6-sialyltransferase (ST), alpha 2,3-sialyltransferase and/or variants thereof operably linked to at least one promoter.
14. Plant or plant cell according to any one of embodiments 1 to 13, wherein the plant or plant cell comprises a nucleic acid sequence encoding for at least one fucosyltransferase, preferably a core alpha 1,6-fucosyltransferase (FUT8), operably linked to at least one promoter and/or a core alpha 1,3-fucosyltransferase operably linked to at least one promoter
15. Plant or plant cell according to any one of embodiments 1 to 14, wherein the plant or plant cell comprises a nucleic acid sequence encoding for at least one N-acetylglucosaminyltransferase, preferably a beta 1,6-N-acetylglucosaminyltransferase (GnTV) or a beta 1,4-N-acetylglucosaminyltransferase (GnTIV), or a beta,1,2-N-acetylglucosaminyltransferase (GnTII) operably linked to at least one promoter.
16. Plant or plant cell according to any one of embodiments 1 to 15, wherein the plant or plant cell comprises at least one nucleic acid sequence encoding for at least one endoglucosaminidase operably linked to a promoter.
17. Plant or plant cell according to embodiment 16, wherein the endoglucosaminidase operably linked to a promoter is an endo-beta-N-acetylglucosaminidase, in particular Endo T.
18. Plant or plant cell according to any one of embodiments 1 to 17, wherein the polysialyltransferase binding motif is a fibronectin type III domain or a FN1 acidic patch, preferably a DEPE motif.
19. Plant or plant cell according to any one of embodiments 1 to 18, wherein the polypeptide lacking a polysialyltransferase binding motif is a glycoprotein.
20. Plant or plant cell according to any one of embodiments 1 to 18, wherein the glycosylation site is a N-glycosylation site or a mucin-type O-glycosylation site.
21. Plant or plant cell according to any one of embodiments 1 to 20, wherein the polypeptide lacking a polysialyltransferase binding motif is selected from the group consisting of antibodies, preferably IgG, IgA, IgM, IgD and IgE and fragments thereof including single chain antibodies (scFvs), heavy chain antibodies, Fab-fragments, nanobodies and Fcabs.
22. Plant or plant cell according to any one of embodiments 1 to 21, wherein the polypeptide lacking a polysialyltransferase binding motif is selected from the group consisting of antigen-binding non-immunoglobulin proteins.
23. Plant or plant cell according to any one of embodiments 1 to 21, wherein the polypeptide lacking a polysialyltransferase binding domain is selected from the group consisting of erythropoietin, α1-Antitrypsin, transferrin, butyrylcholinesterase, granulocyte colony-stimulating factor, DNAse 1, clotting factors, in particular factor VII, factor VIII, factor IX or von Willebrand factor, follicle-stimulating hormone, luteinizing hormone, thyroid-stimulating hormone, interferons, in particular interferon alpha, interferon beta or interferon gamma, tumor necrosis factor-alpha inhibitors, in particular etanercept, viral proteins, viral antigens, and fragments, mutants or variants thereof.
24. Plant or plant cell according to any one of embodiments 1 to 23, wherein the polypeptide lacking a polysialyltransferase binding domain has been modified to introduce a glycosylation site.
25. Plant or plant cell according to embodiment 24, wherein the polypeptide lacking a polysialyltransferase binding domain is insulin.
26. Plant or plant cell according to any one of embodiments 1 to 25, wherein the plant is selected from the group consisting of the genera Nicotiana, Arabidopsis, Lemna, Physcomitrella, Zea, Oryza, Triticum, Pisum, Lotus, Taxus and Brassica or selected from the group consisting of algae safflower, alfalfa, lettuce, barley, rapeseed, soybean, sugar beet, sugar cane, potato, tomato, spinach, ginseng, gingko and carrots and the plant cell is derived from said plants.
27. Plant or plant cell according to any one of embodiments 1 to 26, wherein the plant is selected from the group of plant species consisting of Nicotiana benthamiana, Nicotiana tabacum, *Arabidopsis thaliana, Lemna minor, Physcomitrella patens, Zea mays, Oryza sativa, Triticum aestivum, Pisum sativum, Lotus japonicas, Taxus cuspidate,* and *Brassica napus.*
28. Plant or plant cell according to any one of embodiments 1 to 27, wherein the plant cell is selected from the group consisting of tobacco BY2 cells, carrot cells, medicago cells or rice cells.
29. Plant or plant cell according to any one of embodiments 1 to 28, wherein the plant cell is a cambial meristematic cell.
30. Plant or plant cell according to any one of embodiments 1 to 29 wherein the plant cell is derived from Nicotiana benthamiana leaves.
31. Method for producing a polysialylated polypeptide comprising the step of cultivating a plant or plant cell according to any one of embodiments 1 to 30.
32. Method according to embodiment 31, wherein the plant cell is cultivated in suspension culture.
33. Method according to embodiment 31 or 32, wherein the nucleic acid sequences are introduced into the plant or plant cell by agroinfiltration of the plant cell, plants or parts thereof including leaves.
34. Polysialylated polypeptide obtainable by a method according to any one of embodiments 31 to 33.
35. Polypeptide according to embodiment 34, wherein the polysialylated polypeptide comprises a polysialic acid chain comprising at least 2, preferably at least 4, more preferably at least 8, sialic acid units.
36. Polypeptide according to embodiment 34 or 35, wherein the polysialylated polypeptide comprises a polysialic acid chain comprising 2 to 400, preferably 2 to 300, more preferably 2 to 250, sialic acid units.
37. Use of a plant or plant cell according to any one of embodiments 1 to 30 for producing a polysialylated polypeptide from a polypeptide lacking a polysialyltransferase binding motif and comprising at least one glycosylation site.

### EXAMPLES:

### Example 1: Multi-gene binary vectors for Nicotiana benthamiana stable transformation

The triple gene vectors pC144 and pG371 containing the expression cassettes for the UDP-*N*-acetylglucosamine 2-epimerase/*N*-acetylmannosamine-kinase (GNE), *N-*acetylneuraminic acid phosphate-synthase (NANS), CMP-Neu5Ac synthetase (CMAS), CMP-Neu5Ac transporter (CST), β1,4 - galactosyltransfease fused to the cytoplasmic tail, transmembrane domain and stem region of the α2,6-sialyltransferase (^{ST}GalT) and α2,6-sialyltransferase (ST6) were described previously (Castilho A, et al. PLoS One 8(2013):e54836.). Here these vectors have been modified in order to use them for co-transformation of *N. benthamiana* ΔXTFT glycosylation mutant (Strasser R, et al. Plant Biotechnol J 6(2008):392-402.).

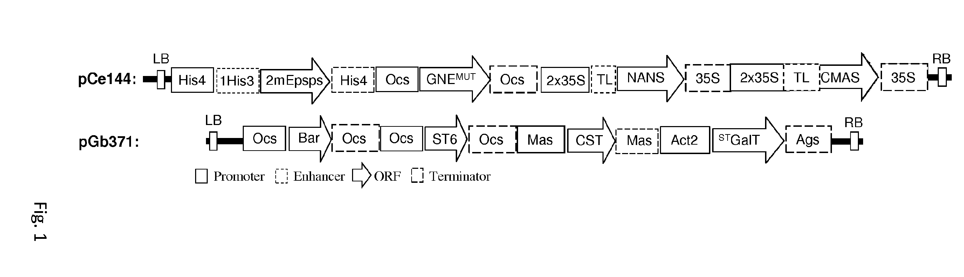

In pC144 an expression cassette encoding glyphosate-resistant 5-enolpyruvoylshikimate-3-phosphate synthase (EPSPS) gene for glyphosate-tolerance was introduced. Annealed Epsps F1/R1 primers (Table 1) were cloned into pC144 to insert *Avr*II*-Nco*I restriction sites. These sites were used to introduce the *epsps* expression cassette as an *Avr*II- *Nco*I fragment (Fig. 1, pCe144).

**Table 1. Primers used in this example.**

| **Primer** | **Restriction** | **Sequence (5'-3'** | **SEQ ID No.** |
|---|---|---|---|
| Epsps F1 | *Avr*II/*Nco*I | cgcgttaatacctaggccatggccat gg | 1 |
| Epsps R1 | *Nco*I/ *Avr*II | cgcgccatggcccatggcctaggtatt aa | 2 |
| GNE^{mut} F | - | agcaaggagatggttctagtgatgcgg aagaag | 3 |
| GNE^{mut} R | - | cttcttccgcatcactagaaccatctc cttgct | 4 |
| *Asc*I^{mut} F | - | ccataaattctagaggcgcatcgcggc cgctcc | 5 |
| *Asc*I^{mut} R. | - | ggagcggccgcatgcgcctctagaat ttatgg | 6 |
| ST3 F1 | *Xba*I | tatatctagaatggtcagcaagtcccg ctggaa | 7 |
| ST3 R1 | *BamH*I | tataggatcctcagaaggacgtgaggt tcttga | 8 |
| ST8Sia-II F1 | XbaI | tatatctagaatgcagctgcagttccg gagc | 9 |
| ST8Sia-II R1 | BglII | tataagatctttacttttcgaactgcg gatggctccacgtggccccatcgcact ggc | 10 |
| ST8Sia-II R2 | BglII | tataagatctcgtggccccatcgcact ggc | 11 |
| ST8Sia-IV F1 | XbaI | tatatctagaatgcgctccattaggaa gag | 12 |
| ST8Sia-IV R1 | *BamH*I | tataggatccttacttttcgaactgcg gatggctccattgctttacacactttc c | 13 |
| ST8Sia-IV R2 | *BamH*I | tataggatccttgctttacacactttc c | 14 |

Restriction sites are in italic and strep-tag sequence in bold.

Also, GNE expression cassette was replaced by a mutated version to prevent feedback inhibition. A point mutation on the GNE gene (R²⁶³L, GNE^{R→L}) was introduced in pSAT1-GNE (Castilho A, et al., PLoS One 8(2013):e54836) using the QuikChange II XL Site-directed Mutagenesis Kit (Strategene, USA) and the primers GNE^{mut} F/R (Table 1), according to manufacturer's instructions. This pSAT1-GNE^{R→L} expression cassette was assembled in pCe144 as AscI-AscI fragment replacing the existing one. Also, an expression cassette for glufosinate ammonium resistance (Basta®) was transferred into pG371 (Fig. 1, pGb371). For this, the Basta resistance cassette was excised from pPZP-RCS2-bar vector (GenBank DQ005454) as *Asc*I-*Asc*I fragment. Since the *Asc*I site was already used to clone in the ST6 expression cassette in pG371 (Castilho A, et al. PLoS One 8(2013):e54836), one of the sites was mutated as described above using the primers *Asc*I^{mut}F/R (Table 1). Both Ce144 and Gb371 constructs were transformed into *Agrobacterium tumefaciens* strain UIA143.

### Example 2: Binary vectors for transient expression of sialyltransferases in N. benthamiana

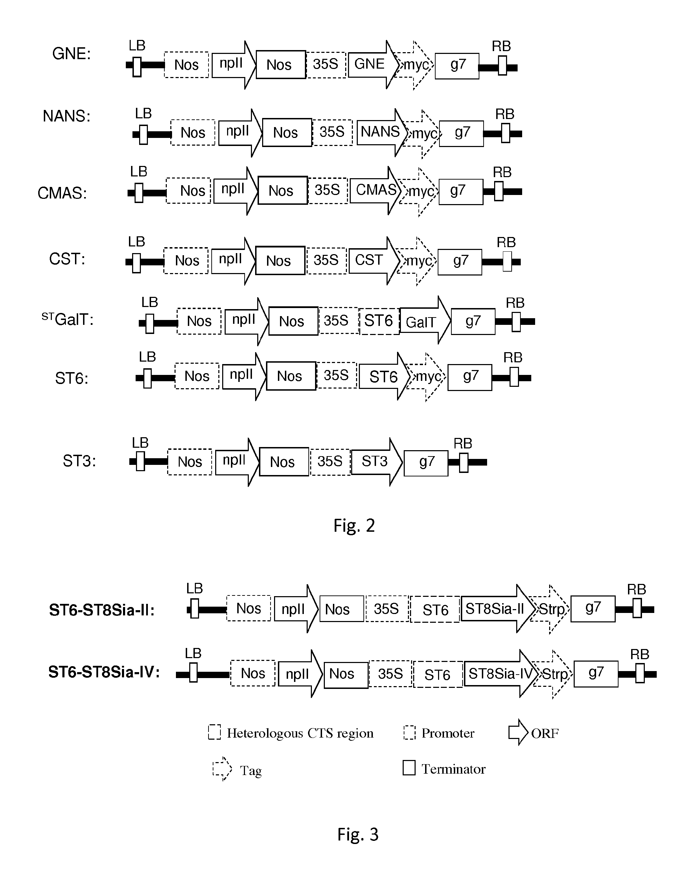

cDNA from the human α2,3-sialyltransferase (IMAGE clone IRAD p970E0336D; Life sciences source bioscience, UK) was PCR amplified with primer pair ST3 F1/R1 (Table 1) and digested with *Xba*I/*Bam*HI and cloned into the binary vector pPT2M (Strasser R, et al. Biochem J 387(2005):385-391) digested the same way. cDNA sequences of two human polysialyltransferases ST8Sia-II (IMAGE clone IRCMp5012E1027D) and ST8Sia-IV (IMAGE clone IRATp970A1079D) (both Life sciences source bioscience, UK) were amplified with a C-terminal Strep II-tag using primer pairs ST8Sia-II F1/R1 and ST8Sia-IV F1/R1 respectively (Table 1). Resulting PCR products were digested with *Xba*I/*Bgl*II (ST8Sia-II) or *Xba*I/*Bam*HI (ST8Sia-IV) and cloned into pPT2M digested with *Xba*I/*Bgl*II or *Xba*I/*Bam*HI. The resulting vectors pST3, pST8Sia-II and pST8Sia-IV (Figs. 2 and 3) were transformed into *A*. *tumefaciens* strain UIA143.

### Example 3: Plant material and plant transformation

*Agrobacterium*-mediated leaf disc transformation of *N. benthamiana* ΔXTFT was performed by a standard protocol (Horsch RB, et al. Science 227(1985):1229-1231). After selection with Basta® (3 mg mL⁻¹) and Glyphosate (200 µM) transgenic plantlets were screened by PCR for the genomic insertion of the 6 mammalian genes. Positive plants were propagated for homozygosity (ΔXTFT^{Sia}). *N. benthamiana* ΔXTFT and ΔXTFT^{Sia} plants were grown in a growth chamber at 22°C with a 16 h light/ 8 h dark photoperiod.

### Example 4: Production of polysialylated glycoproteins

### Material and Methods

### Transient protein expression

Agro-infiltration experiments were carried using four-to-five-week old plants. For modulation of the *N-*glycosylation profiles towards sialylation or polysialylation, recombinant proteins were either expressed in ΔXTFT^{Sia} or co-expressed in ΔXTFT with the necessary constructs (see Kallolimath S et al. (PNAS 2016, doi:10.1073/pnas.1604371113). *Agrobacteria* were infiltrated using optical density (OD₆₀₀) 0.05-0.1. Protein expression was monitored 3-5 days post infiltration.

### Protein extraction and immunoblotting

Total soluble proteins were extracted in 1:2 w/v extraction buffer (100 mM Tris, 1 mM EDTA, 500mM NaCl, 40 mM ascorbic acid). Total proteins were extracted the same way in extract buffer containing 1 % v/v Triton X-100. Secreted proteins were collected from the intracellular fluid (IF) as described previously. Proteins were fractionated in 8 or 12 % SDS-PAGE under reducing conditions and gels were either stained with Coomassie Brilliant Blue or used for immunoblotting. Western blotting was carried out using anti-polySia antibodies (1:750 dilution anti-polysialic acid mAb735). Detection was performed using HRP-conjugated antimouse-IgG A2554, diluted 1:10,000 (Sigma Aldrich, St. Louis, Missouri, USA). Clarity™ Western enhanced chemiluminescence reagents from (Bio-Rad, Life Science, Hercules, California, USA) were used as substrates.

### Results

Various therapeutically interesting proteins (see Fig. 4) were transiently expressed in Nicotiana benthamiana leaves together with the human sialyation pathway and human polysialyltransferases, ST8Sia-II and ST8Sia-IV. Notably reporters do not carry the FN1 domain carrying ST8Sia-II and ST8Sia-IV docking motives or any fragments thereof. Moreover, reporters do not carry any ST8Sia-II or ST8-Sia-IV interacting-regions from other known polysialylated mammalian proteins (like for example the MAM-domain from neuropilin-2 (Bhide et al., JBC, 2016, DOI 10.1074/jbc.M116.714329).

## Claims

1. Plant or plant cell being capable to produce polysialylated glycoproteins comprising at least one recombinant nucleic acid sequence operably linked to a promoter, said recombinant nucleic acid sequence encoding for a polypeptide lacking a polysialyltransferase binding motif and comprising at least one glycosylation site.

2. Plant or plant cell according to claim 1, wherein the plant or plant cell comprises at least one nucleic acid sequence encoding for at least one polysialyltransferase operably linked to at least one promoter.

3. Plant or plant cell according to claim 2, wherein the at least one polysialyltransferase is a alpha2,8-polysialyltransferase.

4. Plant or plant cell according to claim 2 or 3, wherein the at least one polysialyltransferase is selected from the group consisting of ST8Sia-II, ST8Sia-IV and variants thereof.

5. Plant or plant cell according to any one of claims 2 to 4, wherein a cytoplasmic transmembrane stem (CTS) region of the at least one polysialyltransferase is replaced by a heterologous CTS region, preferably by a plant CTS region.

6. Plant or plant cell according to any one of claims 2 to 4, wherein a cytoplasmic transmembrane stem (CTS) region of the at least one polysialyltransferase is replaced by a signal peptide sequence, preferably by a plant signal peptide sequence.

7. Plant or plant cell according to any one of claims 1 to 6, wherein the plant or plant cell comprises nucleic acid sequences encoding for enzymes involved in the synthesis of a sialic acid precursor operably linked to at least one promoter.

8. Plant or plant cell according to any one of claims 1 to 7, wherein genes encoding beta 1,2-xylosyltransferase (XylT) and/or core alpha 1,3-fucosyltransferase (FucT) and/or beta-hexosaminidases (HEXOs) and/or beta 1,3-galactosyltransferases (GALTs) and/or alpha 1,4-fucosyltransferase occurring in the plant or plant cell are mutated, silenced or inactivated to reduce their enzymatic activity within said plant or plant cell.

9. Plant or plant cell according to any one of claims 1 to 8, wherein the plant or plant cell comprises nucleic acid sequences encoding for beta 1,4-galactosyltransfease (GalT), CMP-sialic acid transporter (CST), alpha 2,6-sialyltransferase (ST), alpha 2,3-sialyltransferase and/or variants thereof operably linked to at least one promoter.

10. Plant or plant cell according to any one of claims 1 to 9, wherein the plant or plant cell comprises a nucleic acid sequence encoding for at least one fucosyltransferase, preferably a core alpha 1,6-fucosyltransferase (FUT8), operably linked to at least one promoter and/or a core alpha 1,3-fucosyltransferase operably linked to at least one promoter

11. Plant or plant cell according to any one of claims 1 to 10, wherein the plant or plant cell comprises a nucleic acid sequence encoding for at least one N-acetylglucosaminyltransferase, preferably a beta 1,6-N-acetylglucosaminyltransferase (GnTV) or a beta 1,4-N-acetylglucosaminyltransferase (GnTIV), or a beta,1,2-N-acetylglucosaminyltransferase (GnTII) operably linked to at least one promoter.

12. Plant or plant cell according to any one of claims 1 to 11, wherein the plant or plant cell comprises at least one nucleic acid sequence encoding for at least one endoglucosaminidase operably linked to a promoter.

13. Plant or plant cell according to any one of claims 1 to 12, wherein the polysialyltransferase binding motif is a fibronectin type III domain or a FN1 acidic patch, preferably a DEPE motif.

14. Plant or plant cell according to any one of claims 1 to 13, wherein the plant is selected from the group consisting of the genera Nicotiana, Arabidopsis, Lemna, Physcomitrella, Zea, Oryza, Triticum, Pisum, Lotus, Taxus and Brassica or selected from the group consisting of algae safflower, alfalfa, lettuce, barley, rapeseed, soybean, sugar beet, sugar cane, potato, tomato, spinach, ginseng, gingko and carrots and the plant cell is derived from said plants.

15. Method for producing a polysialylated polypeptide comprising the step of cultivating a plant or plant cell according to any one of claims 1 to 14.

16. Use of a plant or plant cell according to any one of claims 1 to 14 for producing a polysialylated polypeptide from a polypeptide lacking a polysialyltransferase binding motif and comprising at least one glycosylation site.


SEQUENCE LISTING

<110>  Universität für Bodenkultur

<120>  Production of polysialylated polypeptides in plants and plant
       cells

<130>  48237

<160>  38

<170>  PatentIn version 3.5

<210>  1
<211>  29
<212>  DNA
<213>  Artificial Sequence

<220>
<223>  Primer

<400>  1
cgcgttaata cctaggccat gggccatgg                                        29


<210>  2
<211>  29
<212>  DNA
<213>  Artificial Sequence

<220>
<223>  Primer

<400>  2
cgcgccatgg cccatggcct aggtattaa                                        29


<210>  3
<211>  33
<212>  DNA
<213>  Artificial Sequence

<220>
<223>  Primer

<400>  3
agcaaggaga tggttctagt gatgcggaag aag                                   33


<210>  4
<211>  33
<212>  DNA
<213>  Artificial Sequence

<220>
<223>  Primer

<400>  4
cttcttccgc atcactagaa ccatctcctt gct                                   33


<210>  5
<211>  33

<212>  DNA
<213>  Artificial Sequence

<220>
<223>  Primer

<400>  5
ccataaattc tagaggcgca tcgcggccgc tcc                                    33


<210>  6
<211>  33
<212>  DNA
<213>  Artificial Sequence

<220>
<223>  Primer

<400>  6
ggagcggccg cgatgcgcct ctagaattta tgg                                    33


<210>  7
<211>  33
<212>  DNA
<213>  Artificial Sequence

<220>
<223>  Primer

<400>  7
tatatctaga atggtcagca agtcccgctg gaa                                    33


<210>  8
<211>  33
<212>  DNA
<213>  Artificial Sequence

<220>
<223>  Primer

<400>  8
tataggatcc tcagaaggac gtgaggttct tga                                    33


<210>  9
<211>  31
<212>  DNA
<213>  Artificial Sequence

<220>
<223>  Primer

<400>  9
tatatctaga atgcagctgc agttccggag c                                      31


<210>  10
<211>  57
<212>  DNA
<213>  Artificial Sequence

<220>
<223>  Primer

<400>  10
tataagatct ttacttttcg aactgcggat ggctccacgt ggccccatcg cactggc          57


<210>  11
<211>  30
<212>  DNA
<213>  Artificial Sequence

<220>
<223>  Primer

<400>  11
tataagatct cgtggcccca tcgcactggc                                        30


<210>  12
<211>  30
<212>  DNA
<213>  Artificial Sequence

<220>
<223>  Primer

<400>  12
tatatctaga atgcgctcca ttaggaagag                                        30


<210>  13
<211>  55
<212>  DNA
<213>  Artificial Sequence

<220>
<223>  Primer

<400>  13
tataggatcc ttacttttcg aactgcggat ggctccattg ctttacacac tttcc            55


<210>  14
<211>  28
<212>  DNA
<213>  Artificial Sequence

<220>
<223>  Primer

<400>  14
tataggatcc ttgctttaca cactttcc                                          28


<210>  15
<211>  66
<212>  PRT
<213>  Artificial Sequence

<220>
<223>  CTS region

<400>  15

Met Gly Val Phe Ser Asn Leu Arg Gly Pro Lys Ile Gly Leu Thr His
1               5                   10                  15

Glu Glu Leu Pro Val Val Ala Asn Gly Ser Thr Ser Ser Ser Ser Ser
            20                  25                  30

Pro Ser Ser Phe Lys Arg Lys Val Ser Thr Phe Leu Pro Ile Cys Val
        35                  40                  45

Ala Leu Val Val Ile Ile Glu Ile Gly Phe Leu Cys Arg Leu Asp Asn
    50                  55                  60

Ala Ser
65

<210>  16
<211>  66
<212>  PRT
<213>  Artificial Sequence

<220>
<223>  CTS region

<400>  16

Met Gly Val Phe Ser Asn Leu Arg Gly Pro Arg Ala Gly Ala Thr His
1               5                   10                  15

Asp Glu Phe Pro Ala Thr Asn Gly Ser Pro Ser Ser Ser Ser Ser Pro
            20                  25                  30

Ser Ser Ser Ile Lys Arg Lys Leu Ser Asn Leu Leu Pro Leu Cys Val
        35                  40                  45

Ala Leu Val Val Ile Ala Glu Ile Gly Phe Leu Gly Arg Leu Asp Lys
    50                  55                  60

Val Ala
65

<210>  17
<211>  52
<212>  PRT
<213>  Artificial Sequence

<220>
<223>  CTS region

<400>  17

Met Pro Met Arg Tyr Leu Asn Ala Met Ala Ala Leu Leu Met Met Phe

1               5                   10                  15

Phe Thr Leu Leu Ile Leu Ser Phe Thr Gly Ile Leu Glu Phe Pro Ser
            20                  25                  30

Ala Ser Thr Ser Met Glu His Ser Ile Asp Pro Glu Pro Lys Leu Ser
        35                  40                  45

Asp Ser Thr Ser
    50

<210>  18
<211>  90
<212>  PRT
<213>  Artificial Sequence

<220>
<223>  CTS region

<400>  18

Met Ser Lys Arg Asn Pro Lys Ile Leu Lys Ile Phe Leu Tyr Met Leu
1               5                   10                  15

Leu Leu Asn Ser Leu Phe Leu Ile Ile Tyr Phe Val Phe His Ser Ser
            20                  25                  30

Ser Phe Ser Pro Glu Gln Ser Gln Pro Pro His Ile Tyr His Val Ser
        35                  40                  45

Val Asn Asn Gln Ser Ala Ile Gln Lys Pro Trp Pro Ile Leu Pro Ser
    50                  55                  60

Tyr Leu Pro Trp Thr Pro Pro Gln Arg Asn Leu Pro Thr Gly Ser Cys
65                  70                  75                  80

Glu Gly Tyr Phe Gly Asn Gly Phe Thr Lys
                85                  90

<210>  19
<211>  76
<212>  PRT
<213>  Artificial Sequence

<220>
<223>  CTS region

<400>  19

Met Ala Asn Leu Trp Lys Lys Gln Arg Leu Arg Asp Thr Gly Leu Cys
1               5                   10                  15

Arg Leu Gly Ile Leu Phe Ala Val Thr Leu Ser Ile Val Leu Met Leu
            20                  25                  30

Val Ser Val Pro Arg Thr Ala Leu Asn Gly Ser Ser Ile Asp Asp Asp
        35                  40                  45

Leu Asp Gly Leu Asp Lys Asp Leu Glu Ala Lys Leu Asn Ala Ser Leu
    50                  55                  60

Leu Ser Val Ala Arg Gly Asn Arg Met Ser Leu Arg
65                  70                  75

<210>  20
<211>  60
<212>  PRT
<213>  Artificial Sequence

<220>
<223>  CTS region

<400>  20

Met Lys Arg Phe Tyr Gly Gly Leu Leu Val Val Ser Met Cys Met Phe
1               5                   10                  15

Leu Thr Val Tyr Arg Tyr Val Asp Leu Asn Thr Pro Val Glu Lys Pro
            20                  25                  30

Tyr Ile Thr Ala Ala Ala Ser Val Val Val Thr Pro Asn Thr Thr Leu
        35                  40                  45

Pro Met Glu Trp Leu Arg Ile Thr Leu Pro Asp Phe
    50                  55                  60

<210>  21
<211>  118
<212>  PRT
<213>  Artificial Sequence

<220>
<223>  CTS region

<400>  21

Met Lys Gln Phe Met Ser Val Val Arg Phe Lys Phe Gly Phe Thr Ser
1               5                   10                  15

Val Arg Met Arg Asp Trp Ser Val Gly Val Ser Ile Met Val Leu Thr
            20                  25                  30

Leu Ile Phe Ile Ile Arg Tyr Glu Gln Ser Asp His Thr His Thr Val
        35                  40                  45

Asp Asp Ser Ser Ile Glu Gly Glu Ser Val His Glu Pro Ala Lys Lys
    50                  55                  60

Pro His Phe Met Thr Leu Glu Asp Leu Asp Tyr Leu Phe Ser Asn Lys
65                  70                  75                  80

Ser Phe Phe Gly Glu Glu Glu Val Ser Asn Gly Met Leu Val Trp Ser
                85                  90                  95

Arg Met Arg Pro Phe Leu Glu Arg Pro Asp Ala Leu Pro Glu Thr Ala
            100                 105                 110

Gln Gly Ile Glu Glu Ala
        115

<210>  22
<211>  52
<212>  PRT
<213>  Artificial Sequence

<220>
<223>  CTS region

<400>  22

Met Ile His Thr Asn Leu Lys Lys Lys Phe Ser Leu Phe Ile Leu Val
1               5                   10                  15

Phe Leu Leu Phe Ala Val Ile Cys Val Trp Lys Lys Gly Ser Asp Tyr
            20                  25                  30

Glu Ala Leu Thr Leu Gln Ala Lys Glu Phe Gln Met Pro Lys Ser Gln
        35                  40                  45

Glu Lys Val Ala
    50

<210>  23
<211>  51
<212>  PRT
<213>  Artificial Sequence

<220>
<223>  CTS region

<400>  23

Met Ile His Thr Asn Leu Lys Lys Lys Phe Ser Cys Cys Val Leu Val
1               5                   10                  15

Phe Leu Leu Phe Ala Val Ile Cys Val Trp Lys Glu Lys Lys Lys Gly
            20                  25                  30

Ser Tyr Tyr Asp Ser Phe Lys Leu Thr Lys Glu Phe Gln Val Leu Lys
        35                  40                  45

Ser Leu Gly
    50

<210> 24
<211> 52
<212> PRT
<213> Artificial Sequence

<220>
<223> CTS region

<400> 24

Met Val Ser Lys Ser Arg Trp Lys Leu Leu Ala Met Leu Ala Leu Val
1               5                   10                  15

Leu Val Val Met Val Trp Tyr Ser Ile Ser Arg Glu Asp Arg Tyr Ile
            20                  25                  30

Glu Leu Phe Tyr Phe Pro Ile Pro Glu Lys Lys Glu Pro Cys Leu Gln
        35                  40                  45

Gly Glu Ala Glu
    50

<210> 25
<211> 414
<212> PRT
<213> Artificial Sequence

<220>
<223> ST6-ST8Sia-II

<400> 25
   Met Ile His Thr Asn Leu Lys Lys Lys Phe Ser Leu Phe Ile Leu Val
   1               5                   10                  15
   Phe Leu Leu Phe Ala Val Ile Cys Val Trp Lys Lys Gly Ser Asp Tyr
               20                  25                  30
   Glu Ala Leu Thr Leu Gln Ala Lys Glu Phe Gln Met Pro Lys Ser Gln
           35                  40                  45
   Glu Lys Val Ala Leu Glu Asp Ile Ser Glu Ile Glu Glu Glu Ile Gly
       50                  55                  60
   Asn Ser Gly Gly Arg Gly Thr Ile Arg Ser Ala Val Asn Ser Leu His
   65                  70                  75                  80
   Ser Lys Ser Asn Arg Ala Glu Val Val Ile Asn Gly Ser Ser Ser Pro
                   85                  90                  95
   Ala Val Val Asp Arg Ser Asn Glu Ser Ile Lys His Asn Ile Gln Pro
               100                 105                 110
   Ala Ser Ser Lys Trp Arg His Asn Gln Thr Leu Ser Leu Arg Ile Arg
           115                 120                 125
   Lys Gln Ile Leu Lys Phe Leu Asp Ala Glu Lys Asp Ile Ser Val Leu
       130                 135                 140
   Lys Gly Thr Leu Lys Pro Gly Asp Ile Ile His Tyr Ile Phe Asp Arg
   145                 150                 155                 160
   Asp Ser Thr Met Asn Val Ser Gln Asn Leu Tyr Glu Leu Leu Pro Arg
                   165                 170                 175
   Thr Ser Pro Leu Lys Asn Lys His Phe Gly Thr Cys Ala Ile Val Gly
               180                 185                 190
   Asn Ser Gly Val Leu Leu Asn Ser Gly Cys Gly Gln Glu Ile Asp Ala
           195                 200                 205
   His Ser Phe Val Ile Arg Cys Asn Leu Ala Pro Val Gln Glu Tyr Ala
       210                 215                 220
   Arg Asp Val Gly Leu Lys Thr Asp Leu Val Thr Met Asn Pro Ser Val
   225                 230                 235                 240
   Ile Gln Arg Ala Phe Glu Asp Leu Val Asn Ala Thr Trp Arg Glu Lys
                   245                 250                 255
   Leu Leu Gln Arg Leu His Ser Leu Asn Gly Ser Ile Leu Trp Ile Pro
               260                 265                 270
   Ala Phe Met Ala Arg Gly Gly Lys Glu Arg Val Glu Trp Val Asn Glu
           275                 280                 285
   Leu Ile Leu Lys His His Val Asn Val Arg Thr Ala Tyr Pro Ser Leu
       290                 295                 300
   Arg Leu Leu His Ala Val Arg Gly Tyr Trp Leu Thr Asn Lys Val His
   305                 310                 315                 320
   Ile Lys Arg Pro Thr Thr Gly Leu Leu Met Tyr Thr Leu Ala Thr Arg
                   325                 330                 335
   Phe Cys Lys Gln Ile Tyr Leu Tyr Gly Phe Trp Pro Phe Pro Leu Asp
               340                 345                 350
   Gln Asn Gln Asn Pro Val Lys Tyr His Tyr Tyr Asp Ser Leu Lys Tyr
           355                 360                 365
   Gly Tyr Thr Ser Gln Ala Ser Pro His Thr Met Pro Leu Glu Phe Lys
       370                 375                 380
   Ala Leu Lys Ser Leu His Glu Gln Gly Ala Leu Lys Leu Thr Val Gly
   385                 390                 395                 400
   Gln Cys Asp Gly Ala Thr Trp Ser His Pro Gln Phe Glu Lys
                   405                 410

<210>  26
<211>  395
<212>  PRT
<213>  Artificial Sequence

<220>
<223>  ST6-ST8sia-IV

<400>  26

Met Ile His Thr Asn Leu Lys Lys Lys Phe Ser Leu Phe Ile Leu Val
1               5                   10                  15

Phe Leu Leu Phe Ala Val Ile Cys Val Trp Lys Lys Gly Ser Asp Tyr
            20                  25                  30

Glu Ala Leu Thr Leu Gln Ala Lys Glu Phe Gln Met Pro Lys Ser Gln
        35                  40                  45

Glu Lys Val Ala Leu Glu Arg Thr Glu Glu His Gln Glu Thr Gln Leu
    50                  55                  60

Ile Gly Asp Gly Glu Leu Ser Leu Ser Arg Ser Leu Val Asn Ser Ser
65                  70                  75                  80

Asp Lys Ile Ile Arg Lys Ala Gly Ser Ser Ile Phe Gln His Asn Val
                85                  90                  95

Glu Gly Trp Lys Ile Asn Ser Ser Leu Val Leu Glu Ile Arg Lys Asn
            100                 105                 110

Ile Leu Arg Phe Leu Asp Ala Glu Arg Asp Val Ser Val Val Lys Ser

115                 120                 125

Ser Phe Lys Pro Gly Asp Val Ile His Tyr Val Leu Asp Arg Arg Arg
    130                 135                 140

Thr Leu Asn Ile Ser His Asp Leu His Ser Leu Leu Pro Glu Val Ser
145                 150                 155                 160

Pro Met Lys Asn Arg Arg Phe Lys Thr Cys Ala Val Val Gly Asn Ser
                165                 170                 175

Gly Ile Leu Leu Asp Ser Glu Cys Gly Lys Glu Ile Asp Ser His Asn
            180                 185                 190

Phe Val Ile Arg Cys Asn Leu Ala Pro Val Val Glu Phe Ala Ala Asp
        195                 200                 205

Val Gly Thr Lys Ser Asp Phe Ile Thr Met Asn Pro Ser Val Val Gln
    210                 215                 220

Arg Ala Phe Gly Gly Phe Arg Asn Glu Ser Asp Arg Glu Lys Phe Val
225                 230                 235                 240

His Arg Leu Ser Met Leu Asn Asp Ser Val Leu Trp Ile Pro Ala Phe
                245                 250                 255

Met Val Lys Gly Gly Glu Lys His Val Glu Trp Val Asn Ala Leu Ile
            260                 265                 270

Leu Lys Asn Lys Leu Lys Val Arg Thr Ala Tyr Pro Ser Leu Arg Leu
        275                 280                 285

Ile His Ala Val Arg Gly Tyr Trp Leu Thr Asn Lys Val Pro Ile Lys
    290                 295                 300

Arg Pro Ser Thr Gly Leu Leu Met Tyr Thr Leu Ala Thr Arg Phe Cys
305                 310                 315                 320

Asp Glu Ile His Leu Tyr Gly Phe Trp Pro Phe Pro Lys Asp Leu Asn
                325                 330                 335

Gly Lys Ala Val Lys Tyr His Tyr Tyr Asp Asp Leu Lys Tyr Arg Tyr
            340                 345                 350

Phe Ser Asn Ala Ser Pro His Arg Met Pro Leu Glu Phe Lys Thr Leu
        355                 360                 365

Asn Val Leu His Asn Arg Gly Ala Leu Lys Leu Thr Thr Gly Lys Cys
    370                 375                 380

Val Lys Gln Trp Ser His Pro Gln Phe Glu Lys
385                 390                 395

<210>  27
<211>  22
<212>  PRT
<213>  Artificial Sequence

<220>
<223>  Tobacco chitinase signal peptide sequence

<400>  27

Met Lys Thr Asn Leu Phe Leu Phe Leu Ile Phe Ser Leu Leu Leu Ser
1               5                   10                  15

Leu Ser Ser Ala Glu Phe
            20

<210>  28
<211>  20
<212>  PRT
<213>  Artificial Sequence

<220>
<223>  Arabidopsis alpha-glucosidase II signal peptide sequence

<400>  28

Met Arg Ser Leu Leu Phe Val Leu Ser Leu Ile Cys Phe Cys Ser Gln
1               5                   10                  15

Thr Ala Leu Ser
            20

<210>  29
<211>  24
<212>  PRT
<213>  Artificial Sequence

<220>
<223>  Barley alpha-amylase signal peptide sequence

<400>  29

Met Ala Asn Lys His Leu Ser Leu Ser Leu Phe Leu Val Leu Leu Gly
1               5                   10                  15

Leu Ser Ala Ser Leu Ala Ser Gly
            20

<210>  30

<211>  27
<212>  PRT
<213>  Artificial Sequence

<220>
<223>  N. plumbaginifolia calreticulin signal peptide sequence

<400>  30

Met Ala Thr Gln Arg Arg Ala Asn Pro Ser Ser Leu His Leu Ile Thr
1               5                   10                  15

Val Phe Ser Leu Leu Val Ala Val Val Ser Ala
            20                  25

<210>  31
<211>  386
<212>  PRT
<213>  Artificial Sequence

<220>
<223>  Secreted ST8Sia-II

<400>  31

Met Ala Asn Lys His Leu Ser Leu Ser Leu Phe Leu Val Leu Leu Gly
1               5                   10                  15

Leu Ser Ala Ser Leu Ala Ser Gly Asp Ile Ser Glu Ile Glu Glu Glu
            20                  25                  30

Ile Gly Asn Ser Gly Gly Arg Gly Thr Ile Arg Ser Ala Val Asn Ser
        35                  40                  45

Leu His Ser Lys Ser Asn Arg Ala Glu Val Val Ile Asn Gly Ser Ser
    50                  55                  60

Ser Pro Ala Val Val Asp Arg Ser Asn Glu Ser Ile Lys His Asn Ile
65                  70                  75                  80

Gln Pro Ala Ser Ser Lys Trp Arg His Asn Gln Thr Leu Ser Leu Arg
                85                  90                  95

Ile Arg Lys Gln Ile Leu Lys Phe Leu Asp Ala Glu Lys Asp Ile Ser
            100                 105                 110

Val Leu Lys Gly Thr Leu Lys Pro Gly Asp Ile Ile His Tyr Ile Phe
        115                 120                 125

Asp Arg Asp Ser Thr Met Asn Val Ser Gln Asn Leu Tyr Glu Leu Leu
    130                 135                 140

Pro Arg Thr Ser Pro Leu Lys Asn Lys His Phe Gly Thr Cys Ala Ile
145                 150                 155                 160

Val Gly Asn Ser Gly Val Leu Leu Asn Ser Gly Cys Gly Gln Glu Ile
                165                 170                 175

Asp Ala His Ser Phe Val Ile Arg Cys Asn Leu Ala Pro Val Gln Glu
            180                 185                 190

Tyr Ala Arg Asp Val Gly Leu Lys Thr Asp Leu Val Thr Met Asn Pro
        195                 200                 205

Ser Val Ile Gln Arg Ala Phe Glu Asp Leu Val Asn Ala Thr Trp Arg
    210                 215                 220

Glu Lys Leu Leu Gln Arg Leu His Ser Leu Asn Gly Ser Ile Leu Trp
225                 230                 235                 240

Ile Pro Ala Phe Met Ala Arg Gly Gly Lys Glu Arg Val Glu Trp Val
                245                 250                 255

Asn Glu Leu Ile Leu Lys His His Val Asn Val Arg Thr Ala Tyr Pro
            260                 265                 270

Ser Leu Arg Leu Leu His Ala Val Arg Gly Tyr Trp Leu Thr Asn Lys
        275                 280                 285

Val His Ile Lys Arg Pro Thr Thr Gly Leu Leu Met Tyr Thr Leu Ala
    290                 295                 300

Thr Arg Phe Cys Lys Gln Ile Tyr Leu Tyr Gly Phe Trp Pro Phe Pro
305                 310                 315                 320

Leu Asp Gln Asn Gln Asn Pro Val Lys Tyr His Tyr Tyr Asp Ser Leu
                325                 330                 335

Lys Tyr Gly Tyr Thr Ser Gln Ala Ser Ala His Thr Met Pro Leu Glu
            340                 345                 350

Phe Lys Ala Leu Lys Ser Leu His Glu Gln Gly Ala Leu Lys Leu Thr
        355                 360                 365

Val Gly Gln Cys Asp Gly Ala Thr Arg Ser Trp Ser His Pro Gln Phe
    370                 375                 380

Glu Lys
385

<210>  32
<211>  367
<212>  PRT
<213>  Artificial Sequence

<220>
<223>  Secreted ST8Sia-IV

<400>  32

Met Ala Asn Lys His Leu Ser Leu Ser Leu Phe Leu Val Leu Leu Gly
1               5                   10                  15

Leu Ser Ala Ser Leu Ala Ser Gly Arg Thr Glu Glu His Gln Glu Thr
            20                  25                  30

Gln Leu Ile Gly Asp Gly Glu Leu Ser Leu Ser Arg Ser Leu Val Asn
        35                  40                  45

Ser Ser Asp Lys Ile Ile Arg Lys Ala Gly Ser Ser Ile Phe Gln His
    50                  55                  60

Asn Val Glu Gly Trp Lys Ile Asn Ser Ser Leu Val Leu Glu Ile Arg
65                  70                  75                  80

Lys Asn Ile Leu Arg Phe Leu Asp Ala Glu Arg Asp Val Ser Val Val
                85                  90                  95

Lys Ser Ser Phe Lys Pro Gly Asp Val Ile His Tyr Val Leu Asp Arg
            100                 105                 110

Arg Arg Thr Leu Asn Ile Ser His Asp Leu His Ser Leu Leu Pro Glu
        115                 120                 125

Val Ser Pro Met Lys Asn Arg Arg Phe Lys Thr Cys Ala Val Val Gly
    130                 135                 140

Asn Ser Gly Ile Leu Leu Asp Ser Glu Cys Gly Lys Glu Ile Asp Ser
145                 150                 155                 160

His Asn Phe Val Ile Arg Cys Asn Leu Ala Pro Val Val Glu Phe Ala
                165                 170                 175

Ala Asp Val Gly Thr Lys Ser Asp Phe Ile Thr Met Asn Pro Ser Val
            180                 185                 190

Val Gln Arg Ala Phe Gly Gly Phe Arg Asn Glu Ser Asp Arg Glu Lys
        195                 200                 205

Phe Val His Arg Leu Ser Met Leu Asn Asp Ser Val Leu Trp Ile Pro
    210                 215                 220

Ala Phe Met Val Lys Gly Gly Glu Lys His Val Glu Trp Val Asn Ala
225                 230                 235                 240

Leu Ile Leu Lys Asn Lys Leu Lys Val Arg Thr Ala Tyr Pro Ser Leu
                245                 250                 255

Arg Leu Ile His Ala Val Arg Gly Tyr Trp Leu Thr Asn Lys Val Pro
            260                 265                 270

Ile Lys Arg Pro Ser Thr Gly Leu Leu Met Tyr Thr Leu Ala Thr Arg
        275                 280                 285

Phe Cys Asp Glu Ile His Leu Tyr Gly Phe Trp Pro Phe Pro Lys Asp
    290                 295                 300

Leu Asn Gly Lys Ala Val Lys Tyr His Tyr Tyr Asp Asp Leu Lys Tyr
305                 310                 315                 320

Arg Tyr Phe Ser Asn Ala Ser Pro His Arg Met Pro Leu Glu Phe Lys
                325                 330                 335

Thr Leu Asn Val Leu His Asn Arg Gly Ala Leu Lys Leu Thr Thr Gly
            340                 345                 350

Lys Cys Val Lys Gln Gly Ser Trp Ser His Pro Gln Phe Glu Lys
        355                 360                 365

<210>  33
<211>  385
<212>  PRT
<213>  Artificial Sequence

<220>
<223>  ST8Sia-II

<400>  33

Met Gln Leu Gln Phe Arg Ser Trp Met Leu Ala Ala Leu Thr Leu Leu
1               5                   10                  15

Val Val Phe Leu Ile Phe Ala Asp Ile Ser Glu Ile Glu Glu Glu Ile
            20                  25                  30

Gly Asn Ser Gly Gly Arg Gly Thr Ile Arg Ser Ala Val Asn Ser Leu
        35                  40                  45

His Ser Lys Ser Asn Arg Ala Glu Val Val Ile Asn Gly Ser Ser Ser

50                  55                  60

Pro Ala Val Val Asp Arg Ser Asn Glu Ser Ile Lys His Asn Ile Gln
65                  70                  75                  80

Pro Ala Ser Ser Lys Trp Arg His Asn Gln Thr Leu Ser Leu Arg Ile
                85                  90                  95

Arg Lys Gln Ile Leu Lys Phe Leu Asp Ala Glu Lys Asp Ile Ser Val
            100                 105                 110

Leu Lys Gly Thr Leu Lys Pro Gly Asp Ile Ile His Tyr Ile Phe Asp
        115                 120                 125

Arg Asp Ser Thr Met Asn Val Ser Gln Asn Leu Tyr Glu Leu Leu Pro
    130                 135                 140

Arg Thr Ser Pro Leu Lys Asn Lys His Phe Gly Thr Cys Ala Ile Val
145                 150                 155                 160

Gly Asn Ser Gly Val Leu Leu Asn Ser Gly Cys Gly Gln Glu Ile Asp
                165                 170                 175

Ala His Ser Phe Val Ile Arg Cys Asn Leu Ala Pro Val Gln Glu Tyr
            180                 185                 190

Ala Arg Asp Val Gly Leu Lys Thr Asp Leu Val Thr Met Asn Pro Ser
        195                 200                 205

Val Ile Gln Arg Ala Phe Glu Asp Leu Val Asn Ala Thr Trp Arg Glu
    210                 215                 220

Lys Leu Leu Gln Arg Leu His Ser Leu Asn Gly Ser Ile Leu Trp Ile
225                 230                 235                 240

Pro Ala Phe Met Ala Arg Gly Gly Lys Glu Arg Val Glu Trp Val Asn
                245                 250                 255

Glu Leu Ile Leu Lys His His Val Asn Val Arg Thr Ala Tyr Pro Ser
            260                 265                 270

Leu Arg Leu Leu His Ala Val Arg Gly Tyr Trp Leu Thr Asn Lys Val
        275                 280                 285

His Ile Lys Arg Pro Thr Thr Gly Leu Leu Met Tyr Thr Leu Ala Thr
    290                 295                 300

Arg Phe Cys Lys Gln Ile Tyr Leu Tyr Gly Phe Trp Pro Phe Pro Leu
305                 310                 315                 320

Asp Gln Asn Gln Asn Pro Val Lys Tyr His Tyr Tyr Asp Ser Leu Lys
                325                 330                 335

Tyr Gly Tyr Thr Ser Gln Ala Ser Ala His Thr Met Pro Leu Glu Phe
            340                 345                 350

Lys Ala Leu Lys Ser Leu His Glu Gln Gly Ala Leu Lys Leu Thr Val
        355                 360                 365

Gly Gln Cys Asp Gly Ala Thr Arg Ser Trp Ser His Pro Gln Phe Glu
    370                 375                 380

Lys
385

<210>  34
<211>  369
<212>  PRT
<213>  Artificial Sequence

<220>
<223>  ST8Sia-IV

<400>  34

Met Arg Ser Ile Arg Lys Arg Trp Thr Ile Cys Thr Ile Ser Leu Leu
1               5                   10                  15

Leu Ile Phe Tyr Lys Thr Lys Glu Ile Ala Arg Thr Glu Glu His Gln
            20                  25                  30

Glu Thr Gln Leu Ile Gly Asp Gly Glu Leu Ser Leu Ser Arg Ser Leu
        35                  40                  45

Val Asn Ser Ser Asp Lys Ile Ile Arg Lys Ala Gly Ser Ser Ile Phe
    50                  55                  60

Gln His Asn Val Glu Gly Trp Lys Ile Asn Ser Ser Leu Val Leu Glu
65                  70                  75                  80

Ile Arg Lys Asn Ile Leu Arg Phe Leu Asp Ala Glu Arg Asp Val Ser
                85                  90                  95

Val Val Lys Ser Ser Phe Lys Pro Gly Asp Val Ile His Tyr Val Leu
            100                 105                 110

Asp Arg Arg Arg Thr Leu Asn Ile Ser His Asp Leu His Ser Leu Leu

115                 120                 125

Pro Glu Val Ser Pro Met Lys Asn Arg Arg Phe Lys Thr Cys Ala Val
    130                 135                 140

Val Gly Asn Ser Gly Ile Leu Leu Asp Ser Glu Cys Gly Lys Glu Ile
145                 150                 155                 160

Asp Ser His Asn Phe Val Ile Arg Cys Asn Leu Ala Pro Val Val Glu
                165                 170                 175

Phe Ala Ala Asp Val Gly Thr Lys Ser Asp Phe Ile Thr Met Asn Pro
            180                 185                 190

Ser Val Val Gln Arg Ala Phe Gly Gly Phe Arg Asn Glu Ser Asp Arg
        195                 200                 205

Glu Lys Phe Val His Arg Leu Ser Met Leu Asn Asp Ser Val Leu Trp
    210                 215                 220

Ile Pro Ala Phe Met Val Lys Gly Gly Glu Lys His Val Glu Trp Val
225                 230                 235                 240

Asn Ala Leu Ile Leu Lys Asn Lys Leu Lys Val Arg Thr Ala Tyr Pro
                245                 250                 255

Ser Leu Arg Leu Ile His Ala Val Arg Gly Tyr Trp Leu Thr Asn Lys
            260                 265                 270

Val Pro Ile Lys Arg Pro Ser Thr Gly Leu Leu Met Tyr Thr Leu Ala
        275                 280                 285

Thr Arg Phe Cys Asp Glu Ile His Leu Tyr Gly Phe Trp Pro Phe Pro
    290                 295                 300

Lys Asp Leu Asn Gly Lys Ala Val Lys Tyr His Tyr Tyr Asp Asp Leu
305                 310                 315                 320

Lys Tyr Arg Tyr Phe Ser Asn Ala Ser Pro His Arg Met Pro Leu Glu
                325                 330                 335

Phe Lys Thr Leu Asn Val Leu His Asn Arg Gly Ala Leu Lys Leu Thr
            340                 345                 350

Thr Gly Lys Cys Val Lys Gln Gly Ser Trp Ser His Pro Gln Phe Glu
        355                 360                 365

Lys

<210>  35
<211>  529
<212>  PRT
<213>  Artificial Sequence

<220>
<223>  ST6-PSTNmB

<400>  35

Met Ile His Thr Asn Leu Lys Lys Lys Phe Ser Leu Phe Ile Leu Val
1               5                   10                  15

Phe Leu Leu Phe Ala Val Ile Cys Val Trp Lys Lys Gly Ser Asp Tyr
            20                  25                  30

Glu Ala Leu Thr Leu Gln Ala Lys Glu Phe Gln Met Pro Lys Ser Gln
        35                  40                  45

Glu Lys Val Ala Leu Glu Trp Leu Thr Thr Ser Pro Phe Tyr Leu Thr
    50                  55                  60

Pro Pro Arg Asn Asn Leu Phe Val Ile Ser Asn Leu Gly Gln Leu Asn
65                  70                  75                  80

Gln Val Gln Ser Leu Ile Lys Ile Gln Lys Leu Thr Asn Asn Leu Leu
                85                  90                  95

Val Ile Leu Tyr Thr Ser Gln Asn Leu Lys Met Pro Lys Leu Val His
            100                 105                 110

Gln Ser Ala Asn Lys Asn Leu Phe Glu Ser Ile Tyr Leu Phe Glu Leu
        115                 120                 125

Pro Arg Ser Pro Asn Asn Ile Thr Pro Lys Lys Leu Leu Tyr Ile Tyr
    130                 135                 140

Arg Ser Tyr Lys Lys Ile Leu Asn Ile Ile Gln Pro Ala His Leu Tyr
145                 150                 155                 160

Met Leu Ser Phe Thr Gly His Tyr Ser Tyr Leu Ile Ser Ile Ala Lys
                165                 170                 175

Lys Lys Asn Ile Thr Thr His Leu Ile Asp Glu Gly Thr Gly Thr Tyr
            180                 185                 190

Ala Pro Leu Leu Glu Ser Phe Ser Tyr His Pro Thr Lys Leu Glu Arg

195                 200                 205

Tyr Leu Ile Gly Asn Asn Leu Asn Ile Lys Gly Tyr Ile Asp His Phe
    210                 215                 220

Asp Ile Leu His Val Pro Phe Pro Glu Tyr Ala Lys Lys Ile Phe Asn
225                 230                 235                 240

Ala Lys Lys Tyr Asn Arg Phe Phe Ala His Ala Gly Gly Ile Ser Ile
                245                 250                 255

Asn Asn Asn Ile Ala Asn Leu Gln Lys Lys Tyr Gln Ile Ser Lys Asn
            260                 265                 270

Asp Tyr Ile Phe Val Ser Gln Arg Tyr Pro Ile Ser Asp Asp Leu Tyr
        275                 280                 285

Tyr Lys Ser Ile Val Glu Ile Leu Asn Ser Ile Ser Leu Gln Ile Lys
    290                 295                 300

Gly Lys Ile Phe Ile Lys Leu His Pro Lys Glu Met Gly Asn Asn Tyr
305                 310                 315                 320

Val Met Ser Leu Phe Leu Asn Met Val Glu Ile Asn Pro Arg Leu Val
                325                 330                 335

Val Ile Asn Glu Pro Pro Phe Leu Ile Glu Pro Leu Ile Tyr Leu Thr
            340                 345                 350

Asn Pro Lys Gly Ile Ile Gly Leu Ala Ser Ser Ser Leu Ile Tyr Thr
        355                 360                 365

Pro Leu Leu Ser Pro Ser Thr Gln Cys Leu Ser Ile Gly Glu Leu Ile
    370                 375                 380

Ile Asn Leu Ile Gln Lys Tyr Ser Met Val Glu Asn Thr Glu Met Ile
385                 390                 395                 400

Gln Glu His Leu Glu Ile Ile Lys Lys Phe Asn Phe Ile Asn Ile Leu
                405                 410                 415

Asn Asp Leu Asn Gly Val Ile Ser Asn Pro Leu Phe Lys Thr Glu Glu
            420                 425                 430

Thr Phe Glu Thr Leu Leu Lys Ser Ala Glu Phe Ala Tyr Lys Ser Lys
        435                 440                 445

Asn Tyr Phe Gln Ala Ile Phe Tyr Trp Gln Leu Ala Ser Lys Asn Asn
    450                 455                 460

Ile Thr Leu Leu Gly His Lys Ala Leu Trp Tyr Tyr Asn Ala Leu Tyr
465                 470                 475                 480

Asn Val Lys Gln Ile Tyr Lys Met Glu Tyr Ser Asp Ile Phe Tyr Ile
                485                 490                 495

Asp Asn Ile Ser Val Asp Phe His Ser Lys Asp Lys Leu Thr Trp Glu
            500                 505                 510

Lys Ile Lys His Tyr Tyr Tyr Ser Ala Asp Asn Arg Ile Gly Arg Asp
        515                 520                 525

Arg

<210>  36
<211>  528
<212>  PRT
<213>  Artificial Sequence

<220>
<223>  ST8-PSTNmB

<400>  36

Met Arg Ser Ile Arg Lys Arg Trp Thr Ile Cys Thr Ile Ser Leu Leu
1               5                   10                  15

Leu Ile Phe Tyr Lys Thr Lys Glu Ile Ala Arg Thr Glu Glu His Gln
            20                  25                  30

Glu Thr Gln Leu Ile Gly Asp Gly Glu Leu Ser Leu Ser Arg Ser Leu
        35                  40                  45

Val Asn Ser Gly Ser Trp Leu Thr Thr Ser Pro Phe Tyr Leu Thr Pro
    50                  55                  60

Pro Arg Asn Asn Leu Phe Val Ile Ser Asn Leu Gly Gln Leu Asn Gln
65                  70                  75                  80

Val Gln Ser Leu Ile Lys Ile Gln Lys Leu Thr Asn Asn Leu Leu Val
                85                  90                  95

Ile Leu Tyr Thr Ser Gln Asn Leu Lys Met Pro Lys Leu Val His Gln
            100                 105                 110

Ser Ala Asn Lys Asn Leu Phe Glu Ser Ile Tyr Leu Phe Glu Leu Pro

115                 120                 125

Arg Ser Pro Asn Asn Ile Thr Pro Lys Lys Leu Leu Tyr Ile Tyr Arg
    130                 135                 140

Ser Tyr Lys Lys Ile Leu Asn Ile Ile Gln Pro Ala His Leu Tyr Met
145                 150                 155                 160

Leu Ser Phe Thr Gly His Tyr Ser Tyr Leu Ile Ser Ile Ala Lys Lys
                165                 170                 175

Lys Asn Ile Thr Thr His Leu Ile Asp Glu Gly Thr Gly Thr Tyr Ala
            180                 185                 190

Pro Leu Leu Glu Ser Phe Ser Tyr His Pro Thr Lys Leu Glu Arg Tyr
        195                 200                 205

Leu Ile Gly Asn Asn Leu Asn Ile Lys Gly Tyr Ile Asp His Phe Asp
    210                 215                 220

Ile Leu His Val Pro Phe Pro Glu Tyr Ala Lys Lys Ile Phe Asn Ala
225                 230                 235                 240

Lys Lys Tyr Asn Arg Phe Phe Ala His Ala Gly Gly Ile Ser Ile Asn
                245                 250                 255

Asn Asn Ile Ala Asn Leu Gln Lys Lys Tyr Gln Ile Ser Lys Asn Asp
            260                 265                 270

Tyr Ile Phe Val Ser Gln Arg Tyr Pro Ile Ser Asp Asp Leu Tyr Tyr
        275                 280                 285

Lys Ser Ile Val Glu Ile Leu Asn Ser Ile Ser Leu Gln Ile Lys Gly
    290                 295                 300

Lys Ile Phe Ile Lys Leu His Pro Lys Glu Met Gly Asn Asn Tyr Val
305                 310                 315                 320

Met Ser Leu Phe Leu Asn Met Val Glu Ile Asn Pro Arg Leu Val Val
                325                 330                 335

Ile Asn Glu Pro Pro Phe Leu Ile Glu Pro Leu Ile Tyr Leu Thr Asn
            340                 345                 350

Pro Lys Gly Ile Ile Gly Leu Ala Ser Ser Ser Leu Ile Tyr Thr Pro
        355                 360                 365

Leu Leu Ser Pro Ser Thr Gln Cys Leu Ser Ile Gly Glu Leu Ile Ile
    370                 375                 380

Asn Leu Ile Gln Lys Tyr Ser Met Val Glu Asn Thr Glu Met Ile Gln
385                 390                 395                 400

Glu His Leu Glu Ile Ile Lys Lys Phe Asn Phe Ile Asn Ile Leu Asn
                405                 410                 415

Asp Leu Asn Gly Val Ile Ser Asn Pro Leu Phe Lys Thr Glu Glu Thr
            420                 425                 430

Phe Glu Thr Leu Leu Lys Ser Ala Glu Phe Ala Tyr Lys Ser Lys Asn
        435                 440                 445

Tyr Phe Gln Ala Ile Phe Tyr Trp Gln Leu Ala Ser Lys Asn Asn Ile
    450                 455                 460

Thr Leu Leu Gly His Lys Ala Leu Trp Tyr Tyr Asn Ala Leu Tyr Asn
465                 470                 475                 480

Val Lys Gln Ile Tyr Lys Met Glu Tyr Ser Asp Ile Phe Tyr Ile Asp
                485                 490                 495

Asn Ile Ser Val Asp Phe His Ser Lys Asp Lys Leu Thr Trp Glu Lys
            500                 505                 510

Ile Lys His Tyr Tyr Tyr Ser Ala Asp Asn Arg Ile Gly Arg Asp Arg
        515                 520                 525

<210>  37
<211>  314
<212>  PRT
<213>  Artificial Sequence

<220>
<223>  ST6-CstII

<400>  37

Met Ile His Thr Asn Leu Lys Lys Lys Phe Ser Leu Phe Ile Leu Val
1               5                   10                  15

Phe Leu Leu Phe Ala Val Ile Cys Val Trp Lys Lys Gly Ser Asp Tyr
            20                  25                  30

Glu Ala Leu Thr Leu Gln Ala Lys Glu Phe Gln Met Pro Lys Ser Gln
        35                  40                  45

Glu Lys Val Ala Leu Glu Lys Lys Val Ile Ile Ala Gly Asn Gly Pro

50                  55                  60

Ser Leu Lys Glu Ile Asp Tyr Ser Arg Leu Pro Asn Asp Phe Asp Val
65                  70                  75                  80

Phe Arg Cys Asn Gln Phe Tyr Phe Glu Asp Lys Tyr Tyr Leu Gly Lys
                85                  90                  95

Lys Cys Lys Ala Val Phe Tyr Asn Pro Ser Leu Phe Phe Glu Gln Tyr
            100                 105                 110

Tyr Thr Leu Lys His Leu Ile Gln Asn Gln Glu Tyr Glu Thr Glu Leu
        115                 120                 125

Ile Met Cys Ser Asn Tyr Asn Gln Ala His Leu Glu Asn Glu Asn Phe
    130                 135                 140

Val Lys Thr Phe Tyr Asp Tyr Phe Pro Asp Ala His Leu Gly Tyr Asp
145                 150                 155                 160

Phe Phe Lys Gln Leu Lys Asp Phe Asn Ala Tyr Phe Lys Phe His Glu
                165                 170                 175

Ile Tyr Phe Asn Gln Arg Ile Thr Ser Gly Val Tyr Met Cys Ala Val
            180                 185                 190

Ala Ile Ala Leu Gly Tyr Lys Glu Ile Tyr Leu Ser Gly Ile Asp Phe
        195                 200                 205

Tyr Gln Asn Gly Ser Ser Tyr Ala Phe Asp Thr Lys Gln Lys Asn Leu
    210                 215                 220

Leu Lys Leu Ala Pro Asn Phe Lys Asn Asp Asn Ser His Tyr Ile Gly
225                 230                 235                 240

His Ser Lys Asn Thr Asp Ile Lys Ala Leu Glu Phe Leu Glu Lys Thr
                245                 250                 255

Tyr Lys Ile Lys Leu Tyr Cys Leu Cys Pro Asn Ser Leu Leu Ala Asn
            260                 265                 270

Phe Ile Glu Leu Ala Pro Asn Leu Asn Ser Asn Phe Ile Ile Gln Glu
        275                 280                 285

Lys Asn Asn Tyr Thr Lys Asp Ile Leu Ile Pro Ser Ser Glu Ala Tyr
    290                 295                 300

Gly Lys Phe Ser Lys Asn Ile Asn Phe Lys
305                 310

<210>  38
<211>  313
<212>  PRT
<213>  Artificial Sequence

<220>
<223>  ST8-CstII

<400>  38

Met Arg Ser Ile Arg Lys Arg Trp Thr Ile Cys Thr Ile Ser Leu Leu
1               5                   10                  15

Leu Ile Phe Tyr Lys Thr Lys Glu Ile Ala Arg Thr Glu Glu His Gln
            20                  25                  30

Glu Thr Gln Leu Ile Gly Asp Gly Glu Leu Ser Leu Ser Arg Ser Leu
        35                  40                  45

Val Asn Ser Gly Ser Lys Lys Val Ile Ile Ala Gly Asn Gly Pro Ser
    50                  55                  60

Leu Lys Glu Ile Asp Tyr Ser Arg Leu Pro Asn Asp Phe Asp Val Phe
65                  70                  75                  80

Arg Cys Asn Gln Phe Tyr Phe Glu Asp Lys Tyr Tyr Leu Gly Lys Lys
                85                  90                  95

Cys Lys Ala Val Phe Tyr Asn Pro Ser Leu Phe Phe Glu Gln Tyr Tyr
            100                 105                 110

Thr Leu Lys His Leu Ile Gln Asn Gln Glu Tyr Glu Thr Glu Leu Ile
        115                 120                 125

Met Cys Ser Asn Tyr Asn Gln Ala His Leu Glu Asn Glu Asn Phe Val
    130                 135                 140

Lys Thr Phe Tyr Asp Tyr Phe Pro Asp Ala His Leu Gly Tyr Asp Phe
145                 150                 155                 160

Phe Lys Gln Leu Lys Asp Phe Asn Ala Tyr Phe Lys Phe His Glu Ile
                165                 170                 175

Tyr Phe Asn Gln Arg Ile Thr Ser Gly Val Tyr Met Cys Ala Val Ala
            180                 185                 190

Ile Ala Leu Gly Tyr Lys Glu Ile Tyr Leu Ser Gly Ile Asp Phe Tyr

195                 200                 205

Gln Asn Gly Ser Ser Tyr Ala Phe Asp Thr Lys Gln Lys Asn Leu Leu
    210                 215                 220

Lys Leu Ala Pro Asn Phe Lys Asn Asp Asn Ser His Tyr Ile Gly His
225                 230                 235                 240

Ser Lys Asn Thr Asp Ile Lys Ala Leu Glu Phe Leu Glu Lys Thr Tyr
                245                 250                 255

Lys Ile Lys Leu Tyr Cys Leu Cys Pro Asn Ser Leu Leu Ala Asn Phe
            260                 265                 270

Ile Glu Leu Ala Pro Asn Leu Asn Ser Asn Phe Ile Ile Gln Glu Lys
        275                 280                 285

Asn Asn Tyr Thr Lys Asp Ile Leu Ile Pro Ser Ser Glu Ala Tyr Gly
    290                 295                 300

Lys Phe Ser Lys Asn Ile Asn Phe Lys
305                 310